(19) Europäisches Patentamt
European Patent Office
Office européen des brevets

(11) **EP 2 988 823 B1**

(12) **EUROPEAN PATENT SPECIFICATION**

(45) Date of publication and mention
of the grant of the patent:
**11.04.2018 Bulletin 2018/15**

(21) Application number: **14718614.2**

(22) Date of filing: **22.04.2014**

(51) Int Cl.:
*A61N 5/06* (2006.01)     *A61M 21/00* (2006.01)
*G06F 19/00* (2018.01)     *F21S 10/02* (2006.01)
*A61B 5/00* (2006.01)     *H05B 37/02* (2006.01)

(86) International application number:
**PCT/EP2014/058111**

(87) International publication number:
**WO 2014/173883 (30.10.2014 Gazette 2014/44)**

(54) **PERSONALISED LIGHTING CONTROL**

PERSONALISIERTE BELEUCHTUNGSSTEUERUNG

COMMANDE D'ÉCLAIRAGE PERSONNALISÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.04.2013 EP 13164906**

(43) Date of publication of application:
**02.03.2016 Bulletin 2016/09**

(73) Proprietor: **Lighten Aps
4420 Regstrup (DK)**

(72) Inventor: **PEDERSEN, Steen Hvidtfeldt
Hessellund
4040 Jyllinge (DK)**

(74) Representative: **Budde Schou A/S
Hausergade 3
1128 Copenhagen K (DK)**

(56) References cited:
**WO-A2-2012/146256     US-A1- 2012 209 358**

• **HELSON H ET AL: "The role of spectral energy of
source and background color in the pleasantness
of object colors.", APPLIED OPTICS, vol. 9, no. 7,
1 July 1970 (1970-07-01), pages 1513-1562,
XP002712228, ISSN: 0003-6935**

## Description

### Field of the Invention

The present invention relates to a lighting system comprising: for initiating change of a mammal's circadian state or wellness state, comprising at least one computational unit with a memory, a processor, at least one input and at least one output. Further, the lighting system comprises at least one light sensor with spectral and luminosity sensitivity and means for providing information through a connection to said least one input of the computational unit. And which computational unit has computational means that are arranged for collecting, storing, and processing data from at least the light sensor making operational information and comparing said operational information with state information about the mammal's circadian state or well-being state and which computational means compares the operational information and the state information for acti- vating means for generating at least one output signal.

### Background of the Invention

Productivity of humans and in general mammals is essential to the industrial world and the wellbeing of human kind and the world as such. Means for optimising the way that humans and generally mammals use their resources are essential to enable humans and mammals to perform better and more effective. As such machinery and better procedures have proven beneficiary to human kind.

It is well known that the society has organised it self with a work schedule with production of all sorts starting at fixed times and lasting for specified periods.

At the same time it is know that individuals each have their chronotype or circadian type that will be an individual rhythm that in most cases will be more or less out of synch with the daily rhythm of scheduled working hours.

Besides plain efficiency during a work schedule, it is also recognized that lights conditions can improve the overall well-being experienced by the individual user thereby increasing the efficiency or the output of the user.

Usually lamps, or light sources therein, are designed to provide light that enables a human or mammal to see objects. This happens by providing a light source that emits light that directly or via reflections excites classical photoreceptors, rods and cones in the retina in an eye.
Hence an image formed on the retina by the lens in the eye results in that the rods and cones transmits signals to the brain carrying information about the shape and colour of what is seen. This process is almost instant.

Besides, the retina also has a third photoreceptor: the photosensitive ganglion cell, the photosensitive Retinal Ganglion Cells (pRGC), or intrinsically photosensitive Retinal Ganglion Cells (ipRGC).

The pRGC respond to light over time or in a cummulative fashion dissimilar to the classical receptors and the pRGC are not image-forming. As such pRGC provide the brain information about the ambient light intensity and colour.

Although some technological efforts have been made to describe a "circadian pacemaker" these efforts have been overly complex, impractical to implement or merely been system that have been limited to certain limited situations, where they can be used.

On such lighting system for control of a subject's circadian cycle is disclosed in patent application US 2005/0015122. The system gives guidance to a method and a system through application of model based predictive control techniques and variations thereof using optimisation schemes based on cost functions and mathematical models such as the Jewett-Kronauer model of the circadian cycle.

However, the system and control signals used in the system focuses on the light intensity and are deficient with respect to the use of colours.

Moreover, the system and control signals are focused on getting a subject "back to a natural phase" and are deficient with respect to boosts of energy for the well being of a subject.

Also, the system and control signals disclosed do not provide any insight into how to make a functioning lightening environment that can be implemented and also be used as an ordinary lighting system that can be used in rooms in private homes,

offices, factories, or hospitals to mention just a few.

Also, the system and control signals disclosed do not provide any insight into how a light source or lamp can be configured to provide a variable colour, and in particular any insight into how the colour and intensity can be varied essentially continuously either individually or together.

Finally, the system and control signals disclosed do not provide any insight into how to reduce power consumption and subsequently $CO_2$-emissions.

Further, in document WO 2012/146256 a lighting system is described, wherein different parameters of an individual, such as person type ("larks" and "owls") are taken into consideration. The gender of said individual is also taken into consideration but no details regarding the dependence between the lighting system settings and the gender are specified.

## Object of the Invention

It is an object of this invention to provide a system that overcomes any of the mentioned shortcomings of mentioned prior art.

The invention is defined in the claims. Other embodiments are merely exemplary. An object of the present disclosure is to provide a lightening system that enables collecting and processing input about present light conditions surrounding a mammal and in particular a human for generating an output that will alter the light conditions surrounding the mammal thereby providing a better lighting environment and thereby increasing the well-being or "energizing" the mammal and/or reducing the resources such as electricity needed to provide the required light.

By light conditions are primarily understood conditions such as luminosity and colour temperature, but uniformity, stability and smooth transitions etc are also generally understood to be included under light conditions.

An object of the present disclosure is to provide a lamp that produces light that that regulates the circadian rhythm at least partially according to the 24-hour cycle. A further object of the disclosure is to provide a lamp that can contribute to the regulation of the hormone melatonin from the pineal gland.

A further object of the disclosure is to provide a lamp that produces light that will regulate the pupil size.

An object of the present disclosure is to provide lamp to interact with and/or to response to a system that enables collecting and processing input about present light conditions surrounding a mammal and in particular a human for generating an output that will alter the light conditions surrounding the mammal thereby providing a better lighting environment and thereby increasing the well-being or "energizing" the mammal and/or reducing the resources such as electricity needed to provide the required light. An object of the disclosure is a lighting system that is intelligent and/or learning and enabled to regulate light based on a user's previous behaviour.

Objects of the disclosure includes, one or several in combination, to optimise the visual conditions of the user, to increase the quality of the lighting in the vicinity or the area surrounding the user, to improve the physical working environment, to stabilise the users biological rhythms such as the circadian rhythm, to increase the general well- being of the user, to reduce the energy consumption required to maintain adequate or optimised lighting and thereby reducing the emission of greenhouse gasses such as $CO_2$ or particulate matters.

It is a primary object of the present disclosure to provide lighting conditions having a positive influence on the mental state of a person.

## Description of the Invention

Accordingly this is achieved by a lighting system to be disclosed, a lamp or a light source to be disclosed and/or a combination of a lighting system and a lamp or a light to be disclosed.

In particular an object of the disclosure is fulfilled according to a lighting system for initiating change of a mammal's circadian state or well-being state, comprising:

- at least one computational unit with a memory, a processor, at least one input and at least one output
- at least one light sensor with spectral and luminosity sensitivity and means for providing information through a connection to said least one input of the computational unit,

which computational unit has computational means that are arranged for collecting, storing, and processing data from at least the light sensor making operational information and comparing said operational information with state information about the mammal's circadian state or well-being state and which computational means compares the operational information and the state information for activating means for generating at least one output signal at least one connector for communicating with and controlling at least one light source and

- which computational unit is configured to calculate and least one output signal configured to output the setting of at least one light source or lamp to
- a colour temperature range between 2700 - 7000 K;
- an intensity range between 10 to 1000 lumens; and
- a Ra-index greater than 92 at all values of colour temperature and intensity.

Thereby one more objects of the disclosure are achieved.

In particular an object of the disclosure is fulfilled according to a lamp emitting light, comprising:

- a housing configured to hold a
- a reflector configured to reflect and/or direct light from
- a least one light source through

- a diffuser, and

wherein said lamp further comprises a light control unit with at least one connector for communicating with and receiving regulating information from a lighting system as disclosed herein and configured to vary at least one light source so the light emitted from the lamp has

- a colour temperature range between 2700 - 7000 K;
- an intensity range between 10 to 1000 lumens; and
- a Ra-index greater than 92 at all values of colour temperature and intensity.

Thereby one more objects of the disclosure are achieved.

In particular an object of the disclosure is fulfilled according to a kit of a lighting system and a lamp or a light source as disclosed in herein.

In the following separate units, elements or sub-systems will be disclosed.

One aspect of the disclosure is a lighting system for initiating change of a mammal's circadian state or well-being state, comprising:

- at least one computational unit with a memory, a processor, at least one input and at least one output
- at least one light sensor with spectral and luminosity sensitivity and means for providing information through a connection to said least one input of the computational unit,
- which computational unit has computational means that are arranged for collecting, storing, and processing data from at least the light sensor making operational information and comparing said operational information with state information about the mammal's circadian state or well-being state and which computational means compares the operational information and the state information for activating means for generating at least one output signal.

Thereby the lighting system provides a system that affects the circadian rhythm or the well-being of the mammal, which can be a human and described as a personal user.

By dynamically and adaptively regulating the light surrounding the user, the lighting system will contribute to optimise the visual conditions of the user, optimise or better the quality of the lighting at working location, change or stabilise the user's biological day rhythms, increase the overall well-being of the user and the overall health condi-tions, reduce the energy consumption needed to provide the required light and thereby reduces the emission of gasses such as C02.

According to an embodiment of the disclosure, the light sensor is a point or a spot sensor measuring a limited area of a few $mm^2$ or $cm^2$. According to an embodiment of the disclosure, the light sensor measures the ambient light by measuring at least light passing part or a whole sphere and typically a half sphere.

According to an embodiment of the disclosure, the light sensor measures at least part of the frequency spectrum of visible light. Typically the light sensor is sensitive to in the wavelength range between 400 nm and 1200 nm.

According to an embodiment of the disclosure, the light sensor measures the light intensity from parts of lx to thousands of lx or sub-ranges therein.

According to an embodiment of the disclosure, the light sensor has a chromatic sensor and a lumen sensor or the light sensor is an integrated chromatic and lumen sensor.

The connector between the light sensor and the computational unit is capable of transmitting operational data or information from the light sensor to the computational unit. In one embodiment the connector is a cable. In one embodiment the connector is a wireless connection.

According to an embodiment of the disclosure, the light sensor is integrated within the computational unit or attached directly to the computational unit. In an embodiment an optical wave guide guides light to the light sensor with or without a lens arrangement with varying view of angle.

According to an embodiment of the disclosure, the light sensor or the optical wave guide is attached to a holder, which holder can be a pair of glasses or other types of headgear.

According to the disclosure, the computational unit is a micro-processor and associated means for connecting to the light sensor.

According to the disclosure, the computational unit is a multipurpose microcomputer such as a laptop, a handheld computer, a PDA, a PC or alike

According to the disclosure, the computational unit is a phone, such as a so-called smart phone.

According to an embodiment of the disclosure, the computational unit as a display or means for attaching to or communication with a display

According to an embodiment of the disclosure, the computational unit has an I/O-device or means for attaching to or communication with an I/O-device.

According to an embodiment of the disclosure, the computational unit has a touchscreen for display and for retrieving input.

According to an embodiment of the disclosure, the computational unit as computational means such as a CPU, a memory and input/output means for retrieving and sending raw data or information.

It is understood, that the computational means has at least means for comparing at least one data from operational information with at least one data from the state information.

The operational information relates to information retrieved by the lighting system, whilst operating the lighting system in surroundings. The operational information can be information about the background light including data about chromaticity and intensity obtained from the light sensor.

The operational information can vary in time. The computational unit can have means for retrieving, storing and processing operational information that vary in time.

The state information refers to data or information about the state of the mammal, where the state can be an actual state or a desired state. The state information can be pre-stored and stationary data about the personal user.

In one embodiment, the state information is the information that the personal user is a A-type person or a B-type person.

In one embodiment, the state information is the information that the personal user has been travelling from one time zone to another time zone, i.e. might experience jet-lag.

In one embodiment the state information is dynamic and measured continuously.

In one embodiment the state information is the level of melatonin of the personal user.

In one embodiment the state information is the conductivity of part of the mammal's skin.

In one embodiment the state information is a personal user selected input based on predefined selectable options relating to the well-being of the personal user.

In one embodiment there is a slide bar with "very tired" at one end and "very fresh" at the other end. There can be another slide bar with "wake me up" at one end and "put me to sleep" at the other end. Thereby state information is provided to the lighting system.

According to an embodiment, the output signal is an electrical signal generated to drive an indicator with a label that shows "too little light" or "too much light" and instruction to the user to move.

According to an embodiment, the output signal is a signal formed as a text pre-selected from a series of predefined text strings with instructions and displayed on a display or a computer screen. The instructions can be variations in scope and wording from "too much red light - seek bluer light", "too bright red light - turn down red light", etc.

According to an embodiment, the output signal is a stimulus suitable for rewarding or punishing the mammal. As such the output signal can activate a feeding device, said feeding device being able to deliver food, drugs, medicine, stimuli, and alike.

According to an embodiment of the disclosure, the lighting system is special in that the said lighting system further comprises at least one connector for communicating with and controlling at least one light source.

Thereby the lighting system via at least the one connector enables the control of at least one light source's intensity or chromaticity according to the output signal.

In one embodiment, the connector is a wire capable of transmitting a control signal.

In one embodiment, the connector is a wireless connection capable of communicating with light sources with at least one communications standard.

In one embodiment the lighting system is configured for providing an output signal, that will simulate a natural wake-up light, which wake-up light gradually increases in intensity and with an increase in colour temperature from red towards blue.

In one embodiment the lighting system is configured for providing an output signal, that will simulate a natural "go-to-sleep" light, which wake-up light gradually decrease in intensity and with a decrease in colour temperature from blue towards red.

In one embodiment the lighting system is configured for providing an output signal, that is "energy light" with a colour temperature that is blue, which is some 6,500 K and with an intensity of 500-1,000 lumen. Alternatively variations thereof cover shorter or longer time.

According to an embodiment of the disclosure, the lighting system is special in that the lighting system further comprises:

- at least one lights control unit with a connection to said output and with a connection to - at least one light source capable of emitting light at a variable chromaticity and/or luminosity.

The lighting system is a system that can control and optimise lighting surrounding the individual user based on the needs and/or desires of the user's specific needs and situation under a variety of circumstances such as during work and during leisure.

According to an embodiment, at least one lights control unit is integrated with at least one light source.

According to an embodiment, the lights control is an integrated circuitry of a microcomputer and the light source is a computer screen display.

According to an embodiment of the disclosure, the lighting system is special in that the light source consists of at least two light emitters with different chromatic emissions.

Thereby the system is enabled for the light source to emit light with a chromaticity between at least the extreme of at least two light emitters.

According to an embodiment, a light source with three light emitters with tree chromatic emissions is used thereby enabling a light source to cover an area of colour temperatures. In one embodiment, one emitter is red LED, another emitter is a blue LED, and the third emitter is a yellow LED.

In one embodiment a multiplicity of emitters are placed in a pattern forming a light source and controlled by one lights control unit.

In one embodiment identical light emitters are used, but at least one emitter is covered by a coloured layer or coating thereby resulting in a colour shift.

According to an embodiment of the disclosure, the lighting system is special in that the light source has a diffuser for diffusing, mixing or blending light from at least one light emitter.

Thereby the system is enabled for the mixing light from different emitters to form a uniform and pleasant light source.

The diffuser can be sand blasted glass, plastic or alike. The diffuser can be a semitransparent glass or plastic.

According to an embodiment of the disclosure, the lighting system is special in that the lighting system further comprises:

- at least one user interface with a connection to the computational unit for making personal data available to the computational means.

Thereby the user will be able to enter information required for the computational unit to process state information and operational information.

In one embodiment, the user interface is a keyboard with alpha numeric keys for enter- ing instructions.

In one embodiment, the user interface is a touch-screen with either alpha numeric touch-areas or specifically designed touch-areas with pre-defined input areas including sex, age, A-person, B-person.

Further embodiments include scroll bars, knops, buttons that can be used to adjust colour temperature, intensity, timing, pre-stored light profiles and a like that are constant in time or varying over time.

According to an embodiment of the disclosure, the lighting system is special in that the lighting system further comprises:

- at least one computational interface for connecting to at least one second computational unit for retrieving and storing information.

Thereby the embodiment enables the lighting system to access and share information with remote databases or data-streaming devices holding information such as medical data about the user.

The computational interface can be a communications wire such as the USB or variants thereof.

The computational interface can be a wireless connection such as Wi-Fi, Bluetooth, or alike.

According to an embodiment of the disclosure, the lighting system is special in that lighting system further comprises:

- at least one biometrical sensor unit with a connection to the computational unit for providing biometric data to the computational means.

Thereby enabling state information to be acquired and used by the lighting unit.

According to one embodiment, the biometrical sensor is a conductivity sensor with means for contact to the mammal's skin and thereby allowing for information relating to the overall stress level or anxiety level.

In one embodiment, the biometrical sensor is a sensor configured for measuring the blood pressure.

In one embodiment, the biometrical sensor is a sensor configured for measuring the pulse and thereby providing information about activity and over time variations or changes in the overall well-being of the user.

In one embodiment, the biometrical sensor is a sensor configured for measuring at least one level of a hormone or transmitter. The hormone or transmitter can be melatonin thereby providing state information relating to the circadian state of the user.

The hormone or transmitter can be serotonin thereby providing state information relating the level of happiness of the user.

The hormone or transmitter can be Cortisol thereby providing state information relating to the level of stress of the user.

According to an embodiment of the disclosure, the lighting system is special in that the lighting system further comprises:

-    at least one activity sensor unit for providing activity data to the computational means.

Thereby is provided for state information such as activities like sleeping, walking, working, sitting, typing, resting and other types of user activities.

Thereby is provided for operational information such as activities like movements in the environment and the light environment. The operational information can be the actual location of the lighting system and/or the user.

In one embodiment, the activity sensor is an accelerometer configured for measuring the acceleration of the user or the lighting system and thereby providing information about the movement of the user or the lighting system. Thereby is provided for determining activities such as sleeping, resting, walking, running, working, or studying. In one embodiment, the activity sensor is a General Position Sensor (GPS) configured for providing the latitude and longitude and thereby providing a basis for determining the solar position at a given time.

In one embodiment, the activity sensor is a General Position Sensor (GPS) configured for providing the position thereby providing a basis for determining if the user is inside or outside or requesting the actual or forecasted weather conditions on the actual position.

According to an embodiment of the disclosure, the lighting system is special in that the lighting system to the computational unit via a connection further comprises at least one additional sensor or detector unit being a unit such as:

-    a time detector or unit,
-    a location detector or unit, - a work schedule detector or unit,
-    a sleep pattern detector or unit,
-    a daylight detector or unit,
-    a mood detector or unit,
-    a health detector or unit,
-    a date detector or unit,
-    an mammal energy detector or unit, or
-    a connection detector or unit for receiving data or information from an unlisted unit via a protocol.

In one embodiment, the time detector or unit is a clock, which can be a digital clock embedded in the computational unit, an external clock or timer.

In one embodiment, the location detector or unit is a general position system (GPS) or means such as the user interface for input of a location by the user.

In one embodiment, the work schedule detector or unit is a calendar, which can be embedded in the computational unit or an external calendar or scheduler provided in a database stored on a different computer or entered

In one embodiment, the sleep pattern detector or unit is a device configured for measuring rapid eye movement (REM) signals and variations thereof, which device has means and processes for interpreting the signals.

In one embodiment, the sleep pattern detector or unit is a handheld device with an accelerometer to be placed in a contact to the user, which contact can be direct or via a bed, to measure the movements of the user during sleep and further with means configured to categorize the movements according to the depth of the sleep.

In one embodiment, the daylight detector or unit is a device configured to determine whether it is day or night.

In one embodiment, the mood detector or unit is a unit that is configured for determining the mood, being happy, depressed, or alike either by measuring a level of a hor-mone and comparing the level with a stored level or by having a pre-defied mood- indicator to be selected by the user through an interface.

In one embodiment, the health detector or unit is an storage unit with a medical record about the user compiled by a series of medical tests performed by a physician or alike and stored on the storage unit that has a connection to the lighting system.

In one embodiment, the date detector or unit is an electronic calendar.

In one embodiment, the mammal energy detector or unit that is a user interface configured to retrieve input about the mammal's energy levels. This can be done by having a list of pre-defined energy levels, or states, such as "feeling tired", "feeling sleepy", "feeling fresh", "feeling awake" and alike that can be selected by the user through an interface such as a touch- screen.

In one embodiment, the connection detector or unit for receiving data or information from an unlisted unit via a protocol thereby providing connection means or interface means with an open standard of communication protocols or means that will broadcast its communication protocols when connected to the lighting unit.

According to an embodiment of the disclosure, the lighting system is special in that the units or detectors are connected to the computational unit or said units or detectors are embedded in the computational unit.

The disclosure includes variations of configurations of the disclosed sensors, detectors or units so that each sensor, detector or unit can be separate and connected by connection means or integrated as one or more units.

According to an embodiment of the disclosure, the lighting system is special in that the light control units or light sources are embedded in the computational unit.

The disclosure includes variations of configurations of the disclosed control units, light sources, light emitters, or screens so that each control units, light sources, light emitters, or screens can be separate and connected by connection means or integrated as one or more units. Further, a non-therapeutical method for initiating change of a mammal's circadian state or well-being state is disclosed, the non-therapeutical method comprising:

- measuring the intensity and the chromaticity of light surrounding the mammal and providing the measuring as light data,
- collecting, storing, and processing measured light data and making operational information thereof, and
- generating an output signal for initiating change of the mammals surroundings.

Thereby a process is disclosed for altering or changing the circadian state or the well-being state of the mammal, which change results in an increased performance or output of the mammal.

In a particular process, the output signal is an audio-visual signal informing the mammal, being a person, to change the surrounding lights by leaving the present location.

The output signal is based on operational information made by the process as a algorithm such as a summation of weighted light intensities at a given spectral value or range of spectral values as part of measured light data consisting of chromatic and intensity values.

According to one embodiment of the non-therapeutical method as disclosed a non-therapeutical method, further comprising:

- retrieving or storing data about a mammals circadian state or well-being state and making state information thereof
- comparing the operational information and the state information before generating an output based on the comparison.

In a particular process, the state information is provided by the user by entering information about the present state being tired, fresh, awake, sleepy, or similar qualitative information through means for entering information, being an input process though an input device.

According to one embodiment of the non-therapeutical method as disclosed a non-therapeutical method where state information and/or operational information along with the output is stored and by continuously processing or processing by request to memorize and find recurrent outputs that reflect preferred outputs for given situations.

According to a further embodiment of the non-therapeutical method as disclosed a non-therapeutical method where the actual state information and/or operational information is compared with the stored or memorized state information and/or operational information and when these are similar by means of a comparison the preferred stored or memorized output is automatically suggested for approval by the personal user or automatically selected and activated.

Thereby the lighting system is intelligent.

In variations of the intelligent lighting system, the comparison or the processing of the state information or the operational data is done by use of a neural network, fuzzy logic, or so-called intelligent computational means for storing and optimizing or finding individual user patterns within a set of information.

In a particular process, the state information is provided by the user by entering information about a desired state such a desire to become tired, become fresh, wake-up, fall asleep, or alike absolute or relative state through means for entering information, being an input process through an input device.

According to one embodiment of the non-therapeutical methods as disclosed a non-therapeutical method, further comprising:

- varying at least one light source's luminosity or chromaticity, which least one light source is present in the surroundings of the mammal generating an output signal for initiating a change of the mammals surrounding light conditions.

In a particular process the variation in light is a reduction of the intensity and a shift in the colour temperature from bluish to reddish of an arrangement of light emitting diodes is performed to meet a user's desire to go to sleep.

In a particular process the variation in light is an increase of the intensity and a shift in the colour temperature from reddish to bluish of an arrangement of light emitting di-odes is performed to meet a user's desire to wake up.

In a particular process the variation in light is a constant high intensity, say 1,000 lumen, bluish, say 6,500 K, light for a fixed period, say 10 minutes, based on the user's desire to get a boost of energy to feel fresh and energised.

It is understood that a mammal with pupil will perceive light intensity according to the size of the pupil. As such it is well known to the person skilled in the art to see guidance in the literature for a particular mammal to determine the needed light intensity for a particular mammal and/or surrounding of the mammal i.e. setting.

One example towards an extreme is a dark room with a mammal such as an owl with a good night vision and with spectral sensitivity will require a light source with a very low intensity, say a few lumen. One example is a working area with incident natural light of some considerable intensity where the mammal is a human with pupils almost as small as possible and in a situation where the colour temperature needs to be changed towards blue. In this case a light source of one or more light bulbs totalling some 1,000 W installed in a standard European grid with a blue filter will provide a sufficient blue light to match and suffi- ciently change the natural background light.

It is obvious simply add more light sources or remove or switch of light sources or emitters to adjust the light intensity to the right level.

It is natural for the person skilled in the art to experiment with the intensity of the light source and the person skilled in the art will also find it natural to continuously adjust the intensity of the light source.

Likewise the person skilled in the art will also find it natural to use lenses or reflectors to focus or to concentrate light to the field of view of the mammal.

The term "chromaticity" is used to define the perceived colour of the light. In general the chromaticity of the light is referred to and stated as the colour temperature (CT), with a reference to the electromagnetic radiation emitted from a black body at a given temperature in Kelvin, [K]. It is acknowledged that the original definition of colour temperature is based on thermal radiation and that many other light sources based on non thermal radiation exits. Furthermore, a so-called corrected colour temperature (CCT) is often used as the perceived, by a human, radiation form a black-body radiator.

This description acknowledges the presence of different standards and definitions, but also assumes that the person skilled in the art has knowledge of conversions between and amongst different standards. If not the person will readily be enabled by seeking literature explaining ex. conversion amongst, sat RGB-, LAB-, Hue-, CIE-etc. colour spaces.

An object of the disclosure is achieved by a lamp emitting light, comprising:

- a housing configured to hold a
- a reflector configured to reflect and/or direct light from
- a light source through
- a diffuser

where the lamp further comprises a light control unit with at least one connector for communicating with and receiving regulating information from a lighting system and configured to vary the light source so the light emitted from the lamp has

- a colour temperature range between 2700 - 7000 K;
- an intensity range between 10 to 1000 lumens; and - a Ra-index greater than 92 at all values of colour temperature and intensity.

The input from a lighting system is at least a signal requesting a certain colour temperature and an intensity.

Thereby the lamp is capable of producing a light that contributes to regulate the circadian state of a subject. The regulation is done in a simple and effective way by having a single source that can simulate the natural light's variation over a 24-hour cycle and essentially as it varies over a one year cycle. Furthermore, the quality is continuous variable to a high degree. Furthermore the lamp is energy efficient.

The reflector is a geometrical shaped object configured to reflect and direct light through the diffuser. The reflector has a reflective coating that reflects the light.

In an embodiment the reflector has a reflectance above 90 % in the 400 nm - 750 nm wavelength range. One coating to provide such reflectance is a barium sulphate based coating.

Other coatings include diffusive coatings such as GORE (R) Diffuse Reflector or equivalent reflectors. Besides a high and uniform reflectance these diffusive coatings also mix light from different emitters.

The diffuser is generally a matted glass, plastic or equivalent semitransparent material configured to mix light rays different emitters to form a uniform although with some direction light source.

Although the preferred embodiments include a reflector and a diffuser, one embodiment of the lamp is without the diffuser. According to a particular embodiment the lamp is without the reflector.

According to an embodiment the lamp is special in that the light source comprises a plurality of light emitters, preferably light emitting diodes or LEDs.

Thereby the lamp is capable of producing a light that can vary and that is robust against failure of one or a few light emitters.

According to an embodiment of the disclosure, the light control unit is configured to detect the fall-out of at least one light emitter. Furthermore, the light control unit is configured to compensate for such detected fall-out of at least one light emitter, by tuning or regulating the remaining emitters.

In particular using LED makes the lamp energy efficient.

According to an embodiment the lamp is special in that light emitters in the light source are configured on the basis colour mixing of chromaticity binning using a plurality of bins, individually or pre-selected plurality of bins formed to generate a warm white, neutral white, or cool white or combinations thereof.

Thereby the lamp in an efficient way utilises a selected few light emitters in that each light emitter contributes to a partition of the desired colour-space.

The person skilled in the art is guided to a standard such as ANSI C78.377-2008, but the disclosure is not limited to this particular standard as the person skilled in the art will find an equivalent starting point in any other standard.

A partition, a part or a bin of a colour space has a coordinate and a certain extend in the colour space. One such colour space is the CIE 1931 in which each position is de- termined by an x- and y-coordinate.

According to an embodiment the lamp is special in that the light source comprises at least one red, at least one blue, at least one green, at least one Royal blue, at least one cyan, at least Phosphor Converted Amber light emitters in combination with at least a white light emitter.

Thereby providing a colour adjustable light source with tones of white. It is understood that the person skilled in the art will understand from the names of the colours, which wavelengths to start with.

According to an embodiment the lamp is special in that the light source comprises at least one red, at least one green, and at least blue light emitters, or comprises at least one white/yellow and phosphor converted light emitters in combination with at least one blue, and at least a first and a second red light emitter combination with at least a white light emitter.

Thereby providing an alternative colour adjustable light source with tones of white.

According to an embodiment the lamp is special in that the light source comprises at least one emitter having a DWL between 620-630 nm (red), at least one emitter having a DWL between 465-485 nm (blue), at least one emitter having a DWL between 520-535 nm (green), at least one emitter having a DWL between 450-465 nm (Royal blue), at least one emitter having a DWL between 490-520 nm (cyan), at least one emitter having a DWL between 588-592 nm (amber) in combination with at least one emitting light source having a CCT between 2700 K - 7000 K or at least one emit- ting warm white light emitting diode having a CCT between 2600 K - 3700K, at least one emitting neutral white light emitting diode having a CCT between 3700 K- 5000 K, and at least one emitting cold white light emitting diode having a CCT between 5000 K - 10000 K.

Thereby providing a preferred and specific starting point for an intensity and colour temperature variable light source. It is understood the above mentioned wavelengths ranges are approximate and that the person skilled in the art will try to reduce the number of light sources used should it become possible.

The wavelength ranges are approximates and defines ranges for the mentioned colours.

According to an embodiment the lamp is special in that the light source comprises at least one emitter having a DWL between 620-630 nm (red), at least one emitter having a DWL between 465-485nm (blue), at least one emitter having a DWL between 520-535 nm (green) in combination with at least one emitting light source having a CCT between 2700 K - 7000 K or at least one emitting warm white light emitting diode having a CCT between 2600 K - 3700K, at least one emitting neutral white light emitting diode having a CCT between 3700 K- 5000 K, and at least one emitting cold white light emitting diode having a CCT between 5000 K - 10000 K.

According to an embodiment the lamp is special in that the light control unit (9) is configured to vary each light emitter by means of a

- a lookup table,
- programmed functions, or
- an interpolation of said lookup table or programmed functions, or both.

Thereby the lamp has a light control unit that by means of any of the mentioned principles is capable of controlling or regulating each light emitter according to the bins or partitions of the colour space and the location of used light emitters.

As such the person skilled in the art will configure a light control unit, which can be a small processor with a memory and an intelligent unit, to reflect the colour space, the bins and the location of the light emitters in this colour space, and by receiving an input from a lighting system to make the lamp light with a particular intensity and colour temperature lookup pre-stored settings of each light emitter in the light source and provide a control to each light emitters accordingly.

In a similar manner, the light control unit can be configured with a mathematical formular with the same input and resulting in an output that will provide the required setting of each light emitter.

In an embodiment interpolation is done between the tabulated values to obtain a smoother transition between desired points in colour space and intensity.

According to an embodiment the lamp is special in that the lamp further includes at least a second connector for communicating with receiving regulating information via a standard wireless network with a standard protocol such as CAN, a private wireless lamp network for communicating with at least one other lamp, or a wired network with a standard protocol such as CAN.

Thereby the lamp is configured to communicate with a lighting system or another lamp via a network.

According to an embodiment the lamp is special in that said lighting system comprises:

-    at least one computational unit with a memory, a processor, at least one input and at least one output,
-    at least one light sensor with spectral and luminosity sensitivity and means for providing information through a connection to said least one input of the computational unit,
-    which computational unit has computational means that are arranged for collecting, storing, and processing data from at least the light sensor mak- ing operational information and comparing said operational information with state information about the mammal's circadian state or well-being state and which computational means compares the operational information and the state information for activating means for generating at least one output signal.

An object of the disclosure is achieved by a kit of at least one lamp as disclosed and at least one lighting system as disclosed, where said lighting system further comprises at least one user interface with a connection to of the computational unit for making personal data available to the computational means.

Thereby the user can select a pre-stored light sequence that varies in intensity and colour.

According another embodiment the kit is special in that said lighting system further comprises at least one computational interface for connecting to at least one second computational unit for retrieving and storing information.

Thereby information about histories of usage can be stored and possibly be processed to make preferential light sequences that can be re-called and re-run.

An object of the disclosure is achieved by a network of lamps comprising a plurality of lamps as disclosed and at least one lighting system as disclosed where said lamps and least one lighting system are configured in zones, each zone being controlled or regulated by at least one lamp and at least one lighting system.

Thereby an area with different working functions or more users with different require- ments for light can easily be configured and obtained.

It is understood that the lamp as described can be implemented in a variety of forms ranging from small units, say from pixel on a screen that are close to be a small and simple light source to larger unit used in say stadiums, halls or outdoor.

A person skilled in the art will understand or otherwise hereby be guided to understand a lamp broadly and will sometimes find that he is closer to a couple of light emitters than a table lamp and at other times he is closer to an array of powerful spots.

As such the disclosed examples of a network is only illustrative and a person skilled in the art will be enabled to scale and transform the disclosure to other settings including office environments, halls, outdoor areas and arenas.

The primary object is according to a first aspect of the present invention achieved by a non-therapeutical method for providing an output control parameter associated with a specific person for controlling a light output of a lamp adapted to vary said light output by said output control parameter, said light output being characterized by a colour temperature and said control parameter comprising a colour temperature control parameter for controlling the colour temperature of said light output, said non-therapeutical method comprising: providing a current time parameter representing the current local time of the day, providing a type parameter from a range of type parameters comprising at least a parameter representing a first person type and a parameter representing a second person type, providing a gender parameter from a range of gender parameters comprising a parameter representing a male person and a parameter representing a female person, and **in a first alternative** providing a first function being a multivariable function and comprising: a first variable representing the local time, a second variable representing said range of type parameters, and a third variable representing said range of gender parameters as input, a range of colour temperature control parameters as output; said first function defining a first peak and a second peak of said range of colour temperature control parameters with respect to said first variable, said first peak having a first maximum at a first time of the day and a first full width at half maximum of at least 30 minutes, said second peak having a second maximum at a second time of the day that is after said first time of the day and a second full width at half maximum of at least 30 minutes, said first function providing at a fixed value of said third variable an output representing a lower colour temperature at said first time of the day for said first person type than for said second person type and an output representing a higher colour temperature at said second time of the day for said first person type than for said second person type, and said first function further providing at a fixed value of said second variable an output representing a lower colour temperature at said first time of the day and said second time of the day for a male person than for a female person, or **in a second alternative** providing a first function being a multivariable function representing:

$$T(t,\ x,\ y) = (T_0(t) - T_b) \cdot c_{type}(x) \cdot c_{gender}(y) + T_b,$$

where $t$ is a first variable representing the local time, $x$ is a second variable representing said range of type parameters, and $y$ is a third variable representing said range of gender parameters, and said first, second, and third variables constitute an input for said first function, $T(t, x, y)$ is the output of said first function and covers a range of temperature control parameters, $T_0(t)$ is a first base function of the first variable $t$ and defining a first peak and a second peak of said range of colour temperature control, said first peak having a first maximum at a first time of the day and a first full width at half maximum of at least 30 minutes, said second peak having a second maximum at a second time of the day that is after said first time of the day and a second full width at half maximum of at least 30 minutes, $c_{type}(x)$ is a second base function of the second variable x and providing an output corresponding to a lower colour temperature at said first time of the day for said first person type than for said second person type and an output representing a higher colour temperature at said second time of the day for said first person type than for said second person type, $c_{gender}(y)$ is a third base function of the third variable $y$ and providing an output corresponding to a lower colour temperature at said first time of the day and said second time of the day for a male person than for a female person, and $T_b$ is a colour temperature base level that is a positive constant for all values of said first, second, and third variables, and **in both the first and the second alternatives** inputting into said first function said current time parameter as said first variable, said type parameter for said specific person as said second variable, and said gender parameter for said specific person as said third variable to yield said colour temperature control parameter as the output from said first function.

By the light being characterized by a colour temperature is to be understood a non-excluding characterisation, and that the light simultaneously may be characterized alternatively or additionally in other ways, for example by an intensity, a spectrum, a polarization, or a radiance. Local time of the day is understood as encompassing time given as a Coordinated Universal Time (UTC) corrected for daylight saving time (summer/winter time) and the time zone in which the specific person is located. The first person type may correspond to a person of type A, i.e. an early riser as described elsewhere in these specifications. The second person type may correspond to a person of type B, i.e. a late sleeper as described elsewhere in these specifications.

The first function should be understood as encompassing any relation between the input and the permissible output. The first function should not be understood as limited to a single analytical expression. The first function being a multivariable function representing the analytical expression $T(t, x, y)$ should be understood as the first function encompassing all analytical expressions giving qualitative equivalent results as the explicitly defined $T(t, x, y)$, for example a rescaling by multiplying the right hand side by a constant gives a qualitative equivalent result. A full width at half maximum of at least a certain number of minutes should be understood as corresponding to an interval of time with a length of at least the certain number of minutes. By the control parameter being associated with a specific person is to be understand as the control parameter representing information on the specific person, for example person type, gender, and age. The control parameter is understood as simultaneously representing a plurality of pieces of information, for example both the person type and gender.

When the lamp is controlled by the output control parameter, the first function will give two "boosts" of blue light during the day that is adapted to be within the circadian stimulus range of most people, which will have the effect of an increased wellbeing for most persons. The specifying of the person type and the gender further improves the effect.

**In the first alternative**, said first function may define a colour temperature base level representing a positive constant colour temperature over a first range of the local time, and said colour temperature may be independent of said second variable and said third variable over said first range, and said first full width at half maximum may represent the width of said first peak at a colour temperature corresponding to the mean of the colour temperature of said first maximum and the colour temperature at said colour temperature base level, and said second full width at half maximum may represent the width of said second peak at a colour temperature corresponding to the mean of the colour temperature of said second maximum and the colour temperature at said colour temperature base level.

**In the second alternative**, said first base function $T_0(t)$ may be zero or approximately zero over a first range of the local time and said first full width at half maximum may represent the width of said first peak at the middle between said first maximum and $T_b$, and said second full width at half maximum may represent the width of said second peak at the middle between said second maximum and $T_b$.

**In the first alternative**, said first function may define a first depression between said first peak and said second peak at fixed values of said second and third variables, said first depression having a first minimum at a third time of the day and said first function may provide an output at said third time of the day that is the same or approximately the same for all values of said second variable and said third variable.

**In the second alternative**, said first base function $T_0(t)$ may define a first depression between said first peak and said second peak, said first depression having a first minimum at a third time of the day, said second base function $c_{type}(x)$ may provide an output at said third time of the day that is the same or approximately the same for all values of said second variable x, and said third base function $c_{type}(x)$ may provide an output at said third time of the day that is the same or approximately the same for all values of said third variable *y*.

Said first minimum and said colour temperature base level may represent approximately the same colour temperature and/or said first range of the local time may comprise said third time of the day.

Said first time of the day may be before noon according to the local time and/or said second time of the day may be after noon according to the local time. Said first time of the day may be between 7 and 10 local time and/or said second time may be between 12 and 15 local time.

Said first full width at half maximum may be longer than 2 hours and/or said second full width at half maximum may be longer than 1 hour. Said first full width at half maximum may be less than 4 hours and/or said second full width at half maximum may be less than 3 hours.

Said first range of the local time may have a length in the range 1 to 10 hours, 3 to 8 hours 5 to 6 hours, 15 to 30 minutes, 30 minutes to 1 hour, 1 to 2 hours, 2 to 3 hours, 3 to 4 hours, 4 to 5 hours, 5 to 6 hours, 6 to 7 hours, 7 to 8 hours, 9 to 10 hours, 10 to 11 hours, and/or 11 to 12 hours.

Said colour temperature base level may correspond to the minimum colour temperature control parameters of said output. Said colour temperature base level may represent a colour temperature in the range 2300-3700 Kelvin, or in the range 2900-3100 Kelvin. Said first function may represent a colour temperature between 5500 and 6000 Kelvin for said second person type and a female person at said first maximum. Said first function may represent a colour temperature between 4500 and 5000 Kelvin for said first type of person and a male person at said first maximum. Said first function may represent a colour temperature between 5500 and 6000 Kelvin for said first person type and a female person at said second maximum. Said first function may represent a colour temperature between 4500 and 5000 Kelvin for said second person type and a male person at said second maximum.

Said light output may further be characterized by an intensity and said control parameter may further comprise an intensity control parameter for controlling the intensity of said light output, and said non-therapeutical method may further comprise: **in the first alternative** providing a second function comprising: a fourth variable representing the local time, a range of intensity control parameters as output, said second function defining a third peak and a fourth peak of said range of intensity control parameters, said third peak having a third maximum at a fourth time of day and a third full width at half maximum of at least 30 minutes, and said fourth peak having a fourth maximum at a fifth time of the day that is after said fourth time of the day and a fourth full width at half maximum of at least 30 minutes, or **in the second alternative** providing a second function representing:

$$I(t) = I_0(t) \cdot c,$$

where *t* is a fourth variable representing the local time and said fourth variable constitutes an input for said second function, $I(t)$ is the output covering a range of intensity control parameters, $I_0(t)$ is a fourth base function of the fourth variable *t* and defining a third peak and a fourth peak of said range of colour temperature control, said third peak having a third maximum at a fourth time of day and a third full width at half maximum of at least 30 minutes, said second peak having a fourth maximum at a fifth time of the day that is after said fourth time of the day and a fourth full width at half maximum of at least 30 minutes, and c is a correction factor, and **in both the first and the second alternatives** inputting into said second function said current time parameter as said fourth variable to yield said intensity control parameter as the output from said second function.

The second function will improve the effect of "boost" of blue light during the day, which will give an increased wellbeing.

Said first variable and said fourth variable may be the same variable.

Said first peak and said third peak may have the same or approximately the same profile with respect to said first variable and/or said second peak, and said fourth peak may have the same or approximately the same profile with respect to said first variable.

**In the first alternative**, said second function may define an intensity base level representing a positive constant intensity over a second range of the local time, and said third full width at half maximum may represent the width of said third peak at an intensity corresponding to the mean of the intensity of said third maximum and the intensity at said intensity base level, and/or said fourth full width at half maximum may represent the width of said fourth peak at an intensity corresponding to the mean of the intensity of said fourth maximum and the intensity at said intensity base level.

**In the second alternative**, said fourth base $I_0(t)$ function may be constant or approximately constant over a second range of the local time and said third full width at half maximum may represent the width of said third peak at the middle between said third maximum and the minimum of said fourth base function $I_0(t)$, and/or said fourth full width at half maximum may represent the width of said fourth peak at the middle between said fourth maximum and the minimum of said fourth base function $I_0(t)$.

**In the second alternative**, said fourth base function $I_0(t)$ may be equal to or approximately equal to an intensity base level over a second range of the local time, where said intensity base level is a constant; said third full width at half maximum may represent the width of said third peak at the middle between said third maximum and said intensity base level, and/or said fourth full width at half maximum may be representing the width of said fourth peak at the middle between said fourth maximum and said intensity base level.

**In the first alternative**, said second function may define a second depression between said third peak and said fourth peak, said second depression having a second minimum at a sixth time of the day. **In the second alternative**, said fourth base function $I_0(t)$ may define a second depression between said third peak and said fourth peak, said second depression having a first minimum at a sixth time of the day.

Said second range of the local time may have a length in the range of 1 to 10 hours, 3 to 8 hours 5 to 6 hours, 15 to 30 minutes, 30 minutes to 1 hour, 1 to 2 hours, 2 to 3 hours, 3 to 4 hours, 4 to 5 hours, 5 to 6 hours, 6 to 7 hours, 7 to 8 hours, 9 to 10 hours, 10 to 11 hours, 11 to 12 hours, 12 to 13 hours, and/or 13 to 14 hours.

Said first range of local time and said second range of the local time may be the same or approximately the same.

Said first time of the day and said fourth time of the day may be the same or approximately the same time of the day, and/or said second time of the day and said fifth time of the day may be the same or approximately the same time of the day, and/or said third time of the day and said sixth time of the day may be the same or approximately the same time of the day. This will further improve the effect of the "boost" of blue light.

Said second minimum and said intensity base level may represent approximately the same intensity.

Said fourth time of the day may be before noon according to the local time and/or said fifth time of the day may be after noon according to the local time. Said fourth time of the day may be between 7 and 10 local time and/or said fifth time may be between 12 and 15 local time.

Said third full width at half maximum may be longer than 2 hours and/or said fourth full width at half maximum may be longer than 1 hour. Said third full width at half maximum may be less than 4 hours and/or said fourth full width at half maximum may be less than 3 hours.

The non-therapeutical method according to the first aspect of the present invention may further comprise: providing an age parameter from a range of age parameters representing different ages of a person, and **in the first alternative** said second function further being a multivariable function and further comprising: a fifth variable representing said range of age parameters as input, and said second function providing at a fixed value of said fourth variable an output that increases monotonically with said fifth variable, or **in the second alternative** said second function further being a multivariable function representing

$$I(t, z) = I_0(t) \cdot c_{age}(z),$$

where $t$ is said fourth variable representing the local time, and $z$ is a fifth variable representing said range of age parameters, and said fourth and fifth variables constitute said input for said second function, $I(t, z)$ is the output of said second function and covers said range of intensity control parameters, $I_0(t)$ is said fourth base function of the fourth variable t and defining a third peak, and $c_{age}(z)$ is a fifth base function of said fifth variable $z$, said fifth base function constituting said correction factor c and providing an output representing an intensity that increases monotonically with the age of said person, and **in both the first and the second alternatives** said non-therapeutical method further comprising: inputting into said second function said age parameter for said specific person as said fifth variable in addition to said current time parameter as said fourth variable to yield said intensity control parameter as the output from said second function.

With the above function of the fifth base function, the specifying of the age further improves the effect of the "boost" of blue light.

**In the first alternative,** said second function may provide at a fixed value of said fourth variable an output that increases exponentially with said fifth variable. **In the second alternative,** said fifth base function $c_{age}(z)$ may represent:

$$c_{age}(z) = 1/(2^{(13/(z-25))}),$$

where the parameter $z$ is given in the unit of years.

**In the first alternative**, said second function may be constant or approximately constant over a third range of the local time at a fixed value of said fifth variable and said first function may define a fifth peak having a fifth maximum at a seventh time of day and a fifth full width at half maximum of at least 15 minutes, and said third range of the local time may be after said second and/or said fourth time of the day and may comprise said seventh time of the day. **In the second alternative**, said second base function may be constant or approximately constant over a third range of the local time and said first base function may define a fifth peak having a fifth maximum at a seventh time of day and a fifth full width at half maximum of at least 15 minutes, and said third range of the local time may be after said second time of the day and may comprise said seventh time of the day. This will give a "boost" of blue light aimed at increasing the alertness of a person at the end of a working day.

Said seventh time of the day may be between 16 and 18 local time. Said fifth full width at half maximum may be longer than 30 minutes. Said fifth full width at half maximum may be less than 2 hours.

Said third range of the local time may have a length in the range of 30 minutes to 1 hour, 1 to 2 hours, 2 to 3 hours, or 3 to 4 hours.

Said first function may represent a colour temperature between 4500 and 5000 Kelvin for said first person type and a female person at said fifth maximum. Said first function may represent a colour temperature between 3750 and 4250 Kelvin for said second person type and a male person at said fifth maximum.

The primary object is according to a second aspect of the present invention achieved by a non-therapeutical method for providing light for a specific person, said non-therapeutical method comprising: providing a lamp adapted to vary its light output by an output control parameter, and providing said output control parameter by the non-therapeutical method according to the first aspect of the present invention.

The primary object is according to a third aspect of the present invention achieved by a lighting system for providing an output control parameter associated with a specific person for controlling a light output of a lamp adapted to vary said light output by said output control parameter, said lighting system comprising: a computational unit adapted for performing an implementation of the non-therapeutical method according to the first aspect of the present invention for providing said output control parameter, a time detector or unit connected to said computational unit for providing said local time of the day in said implementation, and a user interface connected to said computational unit for providing a type parameter from a range of type parameters comprising at least a parameter representing a first person type and a parameter representing a second person type in said implementation, and for providing a gender parameter from a range of gender parameters comprising a parameter representing a male person and a parameter representing a female person in said implementation.

Said user interface further may be for manually providing an age parameter from a range of age parameters representing different ages of a person in said implementation.

The user interface may for example be a smart phone or a tablet computer with a touch screen and with the ability to communicate with the computational unit, for example via Bluetooth or wireless local area network.

The primary object is according to a fourth aspect of the present invention achieved by a lighting system for providing an output control parameter associated with a specific person for controlling a light output of a lamp adapted to vary said light output by said output control parameter, said light output being characterised by a colour temperature and said control parameter comprising a colour temperature control parameter for controlling the colour temperature of said light output, said lighting system comprising:

a computational unit for providing said output and adapted for performing an implementation of a method providing a current time parameter representing the current local time of the day,
providing a type parameter from a range of type parameters comprising at least a parameter representing a first person type and a parameter representing a second person type,
providing a gender parameter from a range of gender parameters comprising a parameter representing a male person and a parameter representing a female person, and
**in a first alternative** providing a first function being a multivariable function and comprising:

a first variable representing the local time, a second variable representing said range of type parameters, and a third variable representing said range of gender parameters as input,
a range of colour temperature control parameters as output;
said first function defining a first peak and a second peak of said range of colour temperature control parameters with respect to said first variable,
said first peak having a first maximum at a first time of the day and a first full width at half maximum of at least 30 minutes,
said second peak having a second maximum at a second time of the day that is after said first time of the day and a second full width at half maximum of at least 30 minutes,
said first function providing at a fixed value of said third variable an output representing a lower colour temperature at said first time of the day for said first person type than for said second person type and an output representing a higher colour temperature at said second time of the day for said first person type than for said second person type, and
said first function further providing at a fixed value of said second variable an output representing a lower colour temperature at said first time of the day and said second time of the day for a male person than for a female person, or **in a second alternative** providing a first function being a multivariable function representing:

$$T(t, x, y) = (T_0(t) - T_b) \cdot c_{type}(x) \cdot c_{gender}(y) + T_b,$$

where $t$ is a first variable representing the local time, $x$ is a second variable representing said range of type parameters, and $y$ is a third variable representing said range of gender parameters, and said first, second and third variables constitutes an input for said first function,

$T(t, x, y)$ is the output of said first function and covers a range of temperature control parameters,

$T_0(t)$ is a first base function of the first variable $t$ and defining a first peak and a second peak of said range of colour temperature control,

said first peak having a first maximum at a first time of the day and a first full width at half maximum of at least 30 minutes,

said second peak having a second maximum at a second time of the day that is after said first time of the day and a second full width at half maximum of at least 30 minutes,

$c_{type}(x)$ is a second base function of the second variable $x$ and providing an output corresponding to a lower colour temperature at said first time of the day for said first person type than for said second person type and an output representing a higher colour temperature at said second time of the day for said first person type than for said second person type,

$c_{gender}(y)$ is a third base function of the third variable $y$ and providing an output corresponding to a lower colour temperature at said first time of the day and said second time of the day for a male person than for a female person, and

$T_b$ is a colour temperature base level that is a positive constant for all values of said first, second, and third variables,

and **in both the first and the second alternatives** inputting into said first function said current time parameter as said first variable, said type parameter for said specific person as said second variable, and said gender parameter for said specific person as said third variable to yield said colour temperature control parameter as the output from said first function,

said lighting system further comprising:

a time detector or unit connected to said computational unit for providing said local time of the day in said implementation, and

a user interface connected to said computational unit for providing a type parameter from a range of type parameters comprising at least a parameter representing a first person type and a parameter representing a second person type in said implementation, and for providing a gender parameter from a range of gender parameters comprising a parameter representing a male person and a parameter representing a female person in said implementation.

The lighting system according to the fourth aspect of the present invention may further comprise any of the features of the non-therapeutical method according to the first aspect of the present invention in therapeutical as well as non-therapeutical applications.

The primary object is according to a fifth aspect of the present invention achieved by a kit comprising a lamp adapted to vary its light output by an output control parameter and lighting system according to the third aspect of the present invention for providing said output control parameter for controlling the light output of said lamp, said lighting system further being connected to said lamp for communicating said output control parameter to said lamp.

The first, second, third, fourth and fifth aspects of the present invention meeting the primary object of the present invention may comprise any one or more features disclosed in relation to any of the technologies for meeting the other object of the present invention and to the aspects and embodiments related to these technologies. Further, any of the technologies for meeting the other object of the present invention and the aspects and embodiments related to these technologies may comprise any one or more features disclosed in relation to the first, second, third and fourth aspects of the present invention for meeting the primary object of the present invention.

## Description of the Drawing

The invention will be described in relation to the drawings and diagrams, whereon:

Figure 1 shows an embodiment where a lighting system with a computational unit with one input and one output,

figure 2 shows an embodiment where a lighting system further has a light source,

figure 3 shows an embodiment where a lighting system has a user interface for obtaining personal data,

figure 4 shows an embodiment where a lighting system has a biometric sensor for receiving biometric data,

figure 5 shows an embodiment where a lighting system has an activity sensor for receiving activity data about the behaviour of the personal user,

figure 6 shows an embodiment where a lighting system has a multiplicity of sensors or detectors for obtaining data and profiles for a combination of inputs to be processed for generating an output that will alter the background lights,

figure 7 shows an embodiment of a system diagram for data and information retrieval and output for an embodiment of the lighting system,

figure 8 shows a flow diagram of one embodiment of a algorithm that will coordinate the retrieval of data from the sensors, detectors and generate updated outputs to be used for control,

figure 9 shows an embodiment where the lighting system includes at least a hand held device with a user interface and a light source and a light sensor,

figure 10 shows an example of the corrected colour temperature (CCT) during a day,

figure 11 shows an example of the outputs of a lighting system during personal users day including a working day, figure 12 shows an example of the outputs of a lighting system configured and loaded with biometric profiles about an A-person,

figure 13 shows an example of the outputs of a lighting system configured and loaded with biometric profiles about a B-person,

figure 14 shows an embodiment of the lamp with a light source, a reflector and a control unit;

figure 15 shows views an embodiment with the housing and a cooler;

figure 16 shows a view of a reflector with a coating;

figure 17 shows a schematic view of drivers from the control unit to individual light emitters; figure 18 shows an embodiment of multiple lamps arranged to light a room in different zones and communicating with multiple lighting systems;

figure 19 shows time series of light intensity measured by the light sensor, light emitted from the lamp, and the colour temperature of the light from the lamp,

figure 20 shows a presently preferred embodiment of a lighting system according to the present invention,

figure 21A shows a graph representing the first function according to a presently preferred embodiment of the invention,

figure 21B is a blow up of the graph shown if figure 21A,

figure 22 shows a presently preferred embodiment of a second function according to the present invention, and

figure 23 shows a presently preferred embodiment of a fifth base function according to the present invention.

**Detailed description of the drawings**

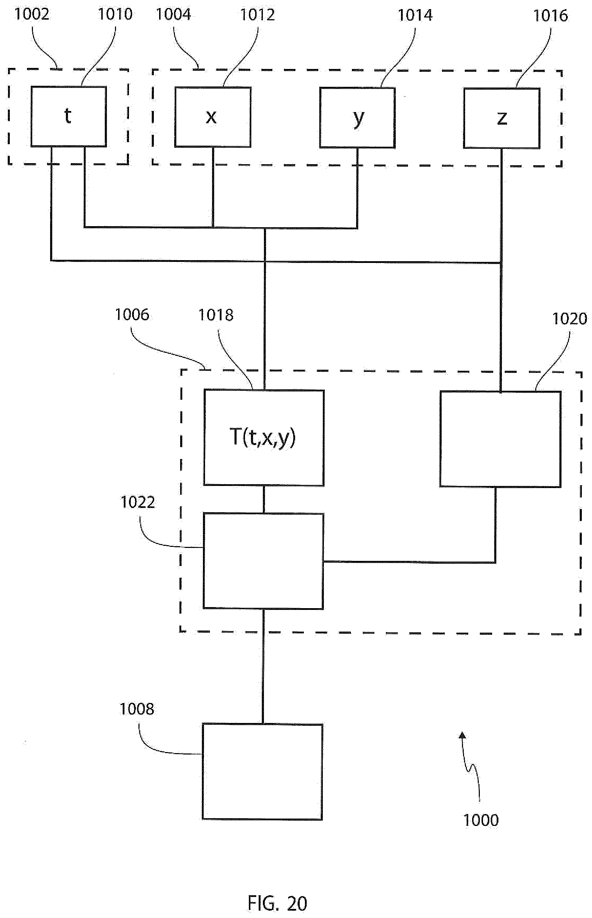

Figure 20 illustrates a presently preferred embodiment of a lighting system 1000. The lighting system 1000 provides an output control parameter for controlling the light output of a lamp 1008 described elsewhere in these specifications. The lighting system 1000 has a computational unit 1006 and a time detector or unit 1002 connected to the computational unit 1006. The time detector or unit 1002 provides the Coordinated Universal Time (UTC) corrected for daylight saving time (summer/winter time) and the time zone in which the lighting system is located as the current time 1010. The time detector or unit 1002 communicates the current time 1010 to the computational unit 1006.

The lighting system 1000 further has a user interface 1004 connected to the computational unit 1006. The user interface 1004 allows the specific user to manually specify if he or she is a person of type A or B (as described elsewhere in these specifications), from which the user interface generates a type parameter 1012. The user interface 1004 then communicates the type parameter 1012 to the computational unit 1006.

The user interface 1004 further allows the specific user to manually specify his or her gender, from which the user interface generates a gender parameter 1014. The user interface 1004 then communicates the gender parameter 1014 to the computational unit 1006. The user interface 1004 further allows the specific user to manually specify his or her age, from which the user interface generates an age parameter 1016. The user interface 1004 then communicates the age parameter 1016 to the computational unit 1006.

The computational unit 1006, which for example can be a tablet computer or a smart phone, is configured to run an algorithm for providing an output control parameter 1022 based on the current time 1010, and type parameter 1012, the gender parameter 1014, and the age parameter 1016. Subsequently, the output control parameter 1022 is communicated to the lamp 1008, which subsequently adjust its output according to the control parameter 1022.

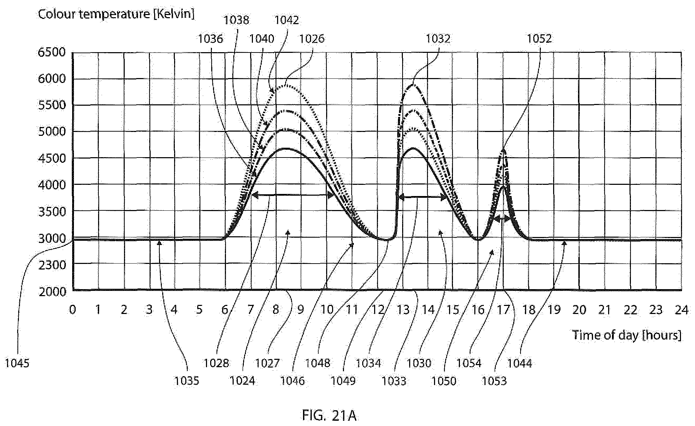

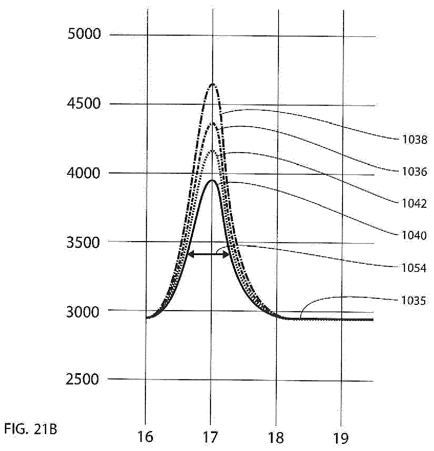

The algorithm represents an implementation of a method for providing an output control parameter in the computational unit 1006. An analytical representation of the algorithm is given below with reference to figures 21 to 23.

A first function 1018 that is a multivariable function is defined as:

$$T(t, x, y) = (T_0(t) - T_b) \cdot c_{type}(x) \cdot c_{gender}(y) + T_b,$$

in which $t$ is a first variable corresponding to the current local time 1010, $x$ is a second variable corresponding to said specified type parameter 1012, and $y$ is a third variable corresponding to the specified gender parameter 1014. With this input, the first function $T(t, x, y)$ gives a single output in the form of a colour temperature control parameter. The colour temperature control parameter constitutes a part of the output control parameter 1022 communicated to the lamp 1008, as is illustrated in figure 20.

$T_0(t)$ is a first base function 1035 of the first variable $t$, which is illustrated in figure 21A-B. The first base function 1035 defines a first peak 1024, a second peak 1030, and a fifth peak 1050. The first peak 1024 has a first maximum 1026 at a first time of the day 1027 and has a first full width at half maximum 1028 that corresponds to a time interval of 3 hours and 15 minutes in length. The second peak 1030 has a second maximum 1032 at a second time of the day 1035 that is after said first time of the day and has a second full width at half maximum 1034 that corresponds to a time interval of 3 hours and 15 minutes in length. The fifth peak 1050 has a fifth maximum 1052 at a seventh time of the day 1053 that is after said first second of the day 1033 and has a fifth full width at half maximum 1054 that corresponds to a time interval of 45 minutes in length.

The first base function 1035 defines a first depression 1046 having a first minimum 1048 at a third time of the day 1049. The first depression 1046 is defined between the first peak 1024 and the second peak 1030.

$c_{type}(x)$ is a second base function of the second variable x. Before 12:30 local time $c_{type}(x)$ gives an output value of 1.0 if a type A person is specified and 1.4 if a type B person is specified. After 12:30 local time $c_{type}(x)$ gives an output value of 1.4 if a type A person is specified and 1.0 if a type B person is specified. $c_{gender}(y)$ is a third base function of the third variable $y$ and gives an output value of 1.0 if the gender is specified as male and 1.2 if the gender is specified as female.

In figures 21A-B, a male person of type A is represented by the curve indexed 1036, a female person of type A is represented by the curve indexed 1038, a male of type B is represented by the curve indexed 1040, a female of type B is represented by the curve indexed 1042. It should be noted that for the first peak 1024, the first base function 1035 and the curve 1036 representing a male person of type A are overlapping and that for the second peak 1030 and the fifth peak 1050, the first base function 1035 and the curve 1040 representing a male person of type B are overlapping.

$T_b$ is a constant with the value 2977 Kelvin representing a colour temperature base level 1045. The first base function is zero for a first range of time 1044 covering a time interval 11 hours in long stretching from 19:00 in the evening to 06:00 in the morning. Consequently, the first function 1018 yields a colour temperature control parameter that is equal to $T_b$ in the first range of time.

A second function 1020 that is a multivariable function is defined as:

$$I(t, z) = I_0(t) \cdot c_{age}(z),$$

in which $t$ is a fourth variable corresponding to the current local time 1010 and, $z$ is a second variable corresponding to the specified age parameter 1016. With this input, the second function $I(t, z)$ gives a single output in the form of an intensity control parameter. The intensity control parameter constitutes a part of the output control parameter 1022 communicated to the lamp 1008, as is illustrated in figure 20.

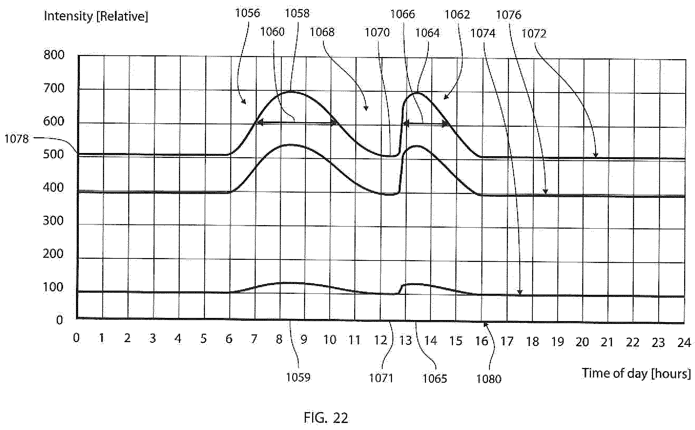

$I_0(t)$ is a fourth base function 1072 of the first variable $t$, which is illustrated in figure 22. The fourth base function 1072 defines a third peak 1056 and a fourth peak 1062. The third peak 1056 has a third maximum 1058 at a fourth time of the day 1059 and has a third full width at half maximum 1060 that corresponds to a time interval of 3 hours and 15 minutes in length. The fourth peak 1062 has a fourth maximum 1064 at a fifth time of the day 1065 that is after said third time of the day and has a fourth full width at half maximum 1066 that corresponds to a time interval of 3 hours and 15 minutes in length.

The fourth base function 1072 defines a second depression 1068 having a second minimum 1070 at a sixth time of the day 1071. The second depression 1068 is defined between the third peak 1056 and the fourth peak 1062. The fourth base function 1072 is constant for a second range of time 1080 covering a time interval 14 hours in long stretching from 16:00 in the afternoon to 06:00 in the morning.

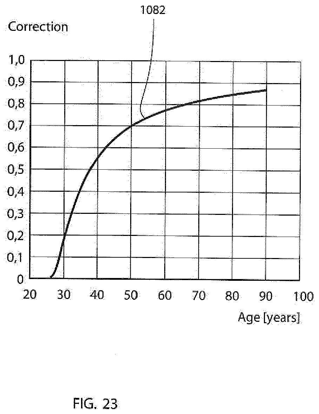

$c_{age}(z)$ is a fifth base function 1082, which is illustrated in figure 23 and is described by the analytical relation:

$$c_{age}(z) = 1/(2^{(13/(z-25))}),$$

where the parameter $z$ is given in the unit of years. The second function 1020 is illustrated in figure 22 and the curve indexed 1074 represents an age of 30 years and the curve indexed 1076 represents an age of 60 years.

The first peak 1024 and the second peak 1030 have the same profile as the third peak 1056 and the fourth peak 1062, respectively. The first time of the day 1027 and the fourth time of the day 1059 correspond to the same local time around 08:30. The second time of the day 1033 and the fifth time of the day 1065 correspond to the same local time around 13:30. The third time of the day 1049 and the sixth time of the day 1071 correspond to the same local time around 12:30. The above means that the colour temperature and intensity are synchronised from 06:00 to 16:00. However, the fourth base function 1072 is constant for a third range of time 1080 covering a time interval of 2 hours in length stretching from 16:00 to 18:00 while the first function 1035 defines the fifth peak in this interval, which means that the colour temperature and intensity are synchronised from 06:00 to 16:00.

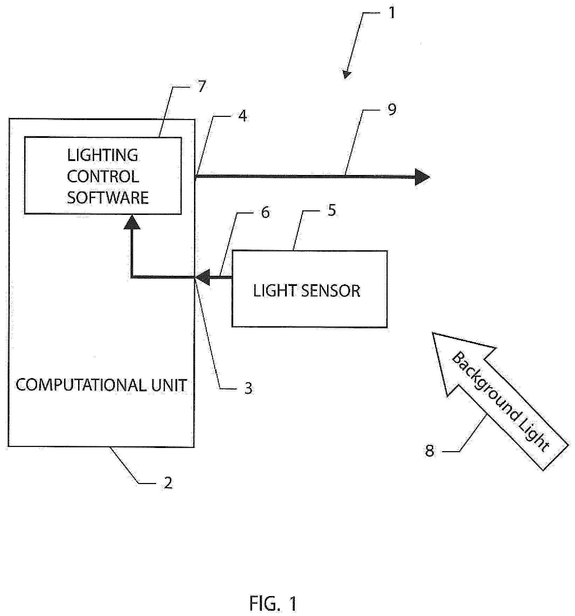

Figure 1 shows an embodiment of the disclosure where a lighting system 1 with a computational unit 2 with one input 3 and one output 4 is shown. The input 3 is connected to light sensor 5 through a connection 6, which in this embodiment is a wire. The light sensor 5 has means for measuring the intensity of the light and means for characterising the chromaticity of the light.

The computational unit 2 has computational means 7 including at least a memory, a central processor and means for storing instructions for collecting, storing and process- ing data from the light sensor 5 and making information and for comparing the light information and comparing said light information with state information about a mammal's light and/or circadian state and which computational means 7 compares the light information and the state information for activating means for generating at least one output signal 4.

The different information types will be described in the following.

The lighting system 1 and the light sensor 5 works in surroundings with background light 8. In an extended version of the embodiment, the lighting system 1 has a connector 9 communicating with at least a light source to control luminance and/or colour temperature.

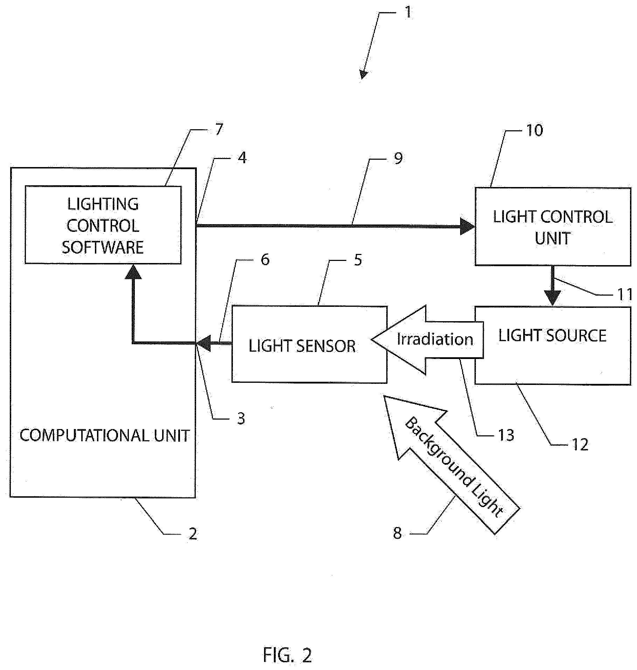

Figure 2 shows an embodiment of the disclosure where a lighting system 1 with a computational unit 2 and a light sensor 5 as seen in figure 1 is show. This embodiment furthermore has the output 4 from the computational unit 2 communicating via a connection 9 to a light control unit 10 that has a connection 1 1 to a light source 12 that is capable of emitting light for irradiation 13 of a mammal mixed with the background light 8 to be collected or measured by the light sensor 5 connected to the input 3 of the computational unit 2.

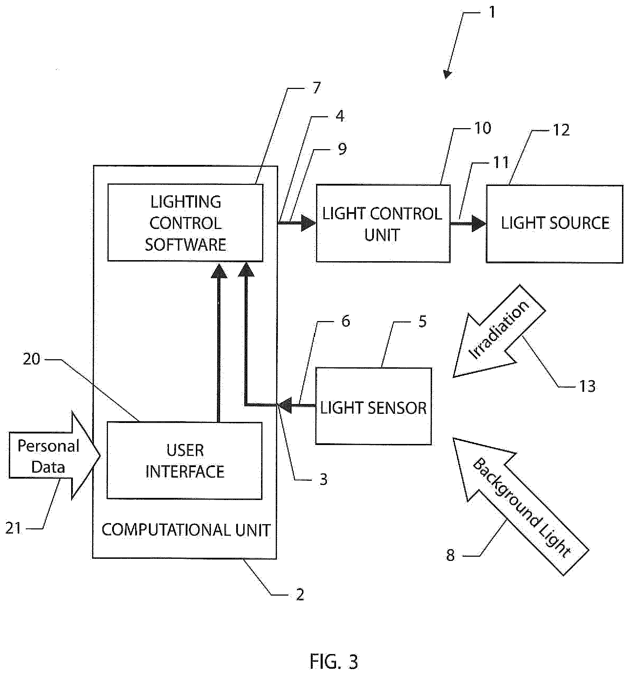

Figure 3 shows an embodiment of the disclosure where a lighting system 1 with a computational unit 2, a light sensor 5, a light control unit 10 and a light source 12 as shown in figure 1 or 2 is shown.

This embodiment furthermore has a user interface 20 embedded in the computational unit 2, which user interface 20 is configured for receiving personal data 21. The computational unit 2 has a memory, processing and editing means for preparing the personal data 21 for adaptation and use by the computational means 7.

Thereby personal data 21 such as information about a persons type, being A-person, Epperson, sex, age etc., can be processed and compared by the input 3 from the light sensor 5 by the computational means 3 for generating a output 4 that via the light control unit 10 makes the light source 12 emit light with a luminance and chromaticity that mixed with the background light 8 to alter the light conditions surrounding the person thereby altering the circadian state of the person accordingly.

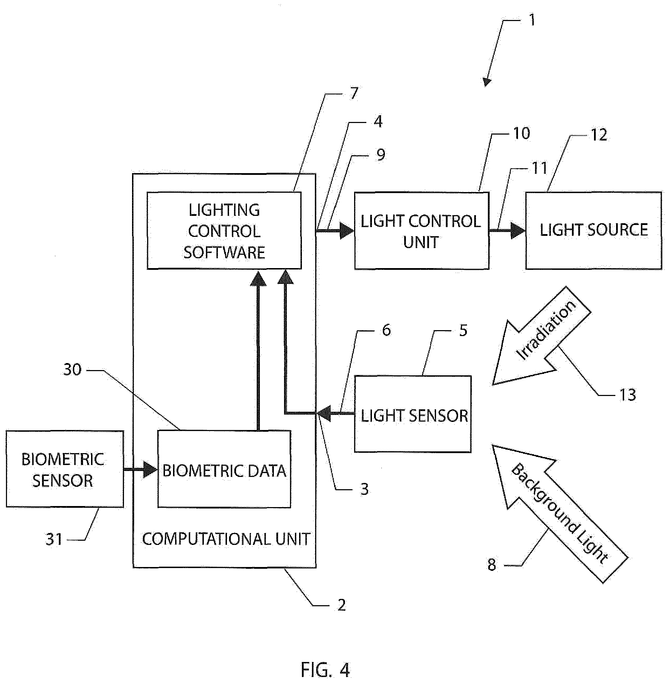

Figure 4 shows an embodiment of the disclosure where a lighting system 1 with a computational unit 2, a light sensor 5, a light control unit 10 and a light source 12 as shown in figure 1, 2 or 3 is shown.

This embodiment furthermore has a biometric sensor 31 connected the computational unit 2 for providing biometric data 30 that can be stored, processed and compared with input 3 from the light sensor 5 by the computational means 7.

Thereby biometric data 30 can be used to generate an output 4 that via the light control unit 10 makes the light source 12 emit light with a luminance and chromaticity that mixed with the background light 8 alters the light conditions surrounding the person thereby altering the circadian state of the person accordingly.

The biometric sensor 31 can have means for measuring the level of melatonin in a per- son or other physical data from which the person's circadian state can be derived.

In a example of the shown embodiment, the computational unit 2 and the computational means 7 are loaded with instructions to accumulate (sum) the light intensity weighted level of blue light (at around 430 nm) over a period of time no longer than a day and produce a "go" output if the accumulated resulting value is below a certain predefined threshold value and to produce a "stay" output if the accumulated resulting value is above the threshold value.

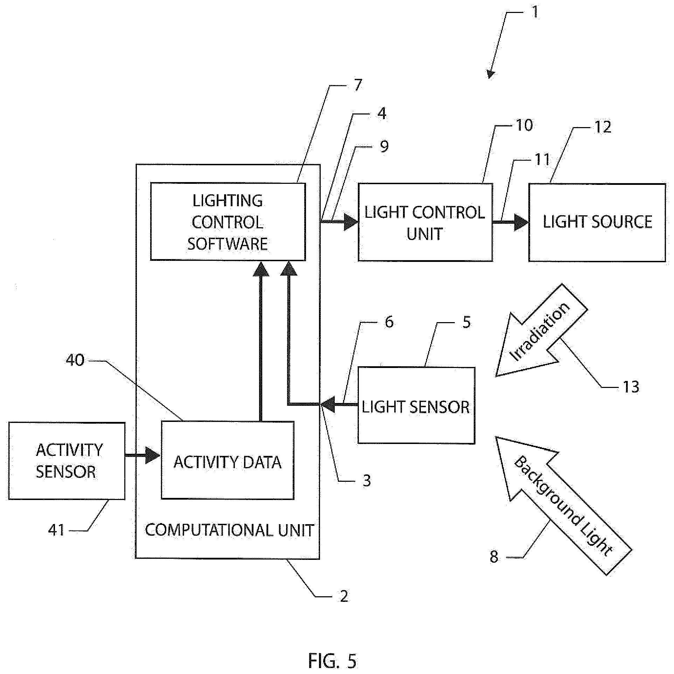

Figure 5 shows an embodiment of the disclosure where a lighting system 1 with a computational unit 2, a light sensor 5, a light control unit 10 and a light source 12 as shown in figure 1, 2, 3 or 4 is shown.

This embodiment furthermore has an activity sensor 41 connected to the computational unit 2 for providing activity data 40 that can be stored, processed and compared with input 3 from the light sensor 5 by the computational means 7.

Thereby activity data 40 can be used to generate an output 4 that via the light control unit 10 makes the light source 12 emit light with a luminance and a chromaticity that mixed with the background light 8 alters the light conditions surrounding the person thereby altering the circadian state of the person accordingly.

The activity sensor 41 can have means for detecting movements, such as one or more accelerometers, of a person and the activity sensor 41, may have pre-processing capabilities to determine if person is running, walking, sitting, working, typing, lying or sleeping just to mention a few, but not limited to these, activities.

In one embodiment, the activity sensor 41 is an accelerometer configured to work with processing system for determining a sleep-wake-cycle.

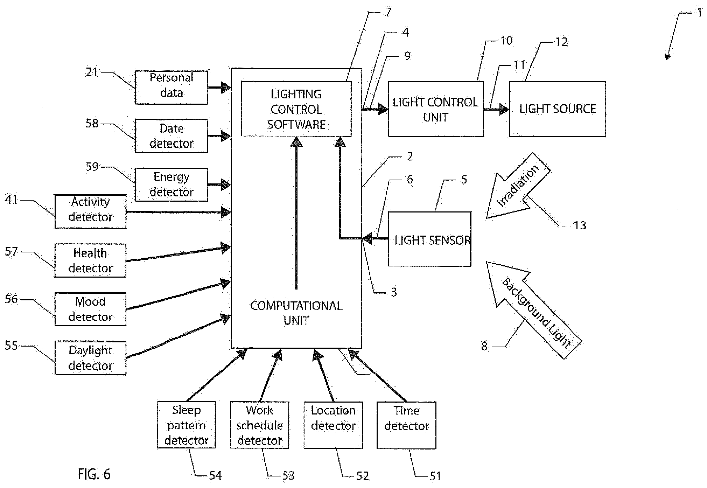

Figure 6 shows an embodiment of the disclosure where a lighting system 1 with a computational unit 2, a light sensor 5, a light control unit 10 and a light source 12 as shown in figure 1, 2, 3, 4 or 5 is shown.

This embodiment furthermore has a multiplicity of detectors or sensor units connected to the computational unit 2 for providing data that can be stored, processed and compared with input 3 from the light sensor 5 by the computational means 7.

The detectors or sensor units connected to the computational unit include a time detector 51, which in this embodiment is a clock.

Further included is a location detector 52, which in this embodiment is a General Position System (GPS).

Further included is a work schedule detector 53, which in this embodiment is an electronic calendar.

Further included is a sleep pattern detector 54, which in this embodiment is an elec- trode for attachment to the mammal for detecting biometrical rhythms such as cardiac rhythms. Further included is a daylight detector 55, which in this embodiment is a signal obtained from an local weather station or a national weather forecasting system.

Further included is a mood detector 56, which in this embodiment is a pad with an sen- sory element for measuring the conductivity of a surface area of the mammal.

Further included is a health detector 57, which in this embodiment is a connection and a protocol for receiving data from the mammals medical or physical record stored in a database.

Further included is a date detector 58, which in this embodiment is a connection and a protocol for receiving data from a calendar with information about the date and the solar position.

Further included is an energy detector 59, which in this embodiment is an interactive display with subjective levels of feelings of or perceived energy levels of the mammal to be entered.

Thereby data from the detectors can be used to generate an output 4 that via the light control unit 10 makes the light source 12 emit light with a luminance and a chroma- ticity that mixed with the background light 8 alters the light conditions surrounding the person thereby altering the circadian state of the person accordingly.

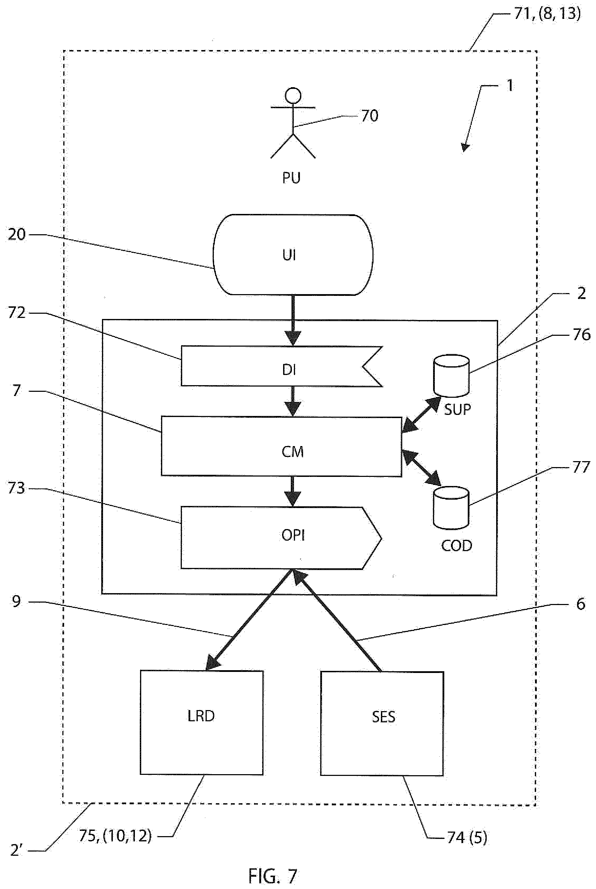

Figure 7 shows an embodiment of a system diagram for data and information retrieval and output for an embodiment of the lighting system 1.

The lighting system 1 works or operates the vicinity of a mammal 70 represented here as a personal user (PU) 70 in a surrounding 71 that is illuminated by background light 8 and irradiation 13 from the lighting system 1. As such the surrounding 71 is the vicinity of the personal user 70 and generally in the field of view of the personal user.

In this embodiment the lighting system has a user interface 20 that has a connection to a data interface (DI) 72 in the computational unit 2 that communicates with the compu-tational means (CM) 7, which again communicates with an operational parameter interface (OPI) 73.

The CM 7 is configured for communicating, storing and receiving data in at least one state user profile SUP 76 memory or structured memory forming a database. The CM 7 is further configured for communicating, storing and receiving data and information in at least one collected operational database (COD) 77.

The OPI 73 is configured for receiving data from surrounding environment sensors SES 74, which in this embodiment is the light sensor 5, but in general a multiplicity of sensors or detectors such as those shown and described previously and in figure 6.

The OPI 73 is further connected to a light radiating device (LRD) 75, which is an integrated unit consisting of a light source 12 and a light control unit 10.

The LRD 75 is capable of lighting the surrounding 71 based on input from the PU 70.

In one obvious variant embodiment of the lighting system 1, the computational unit 2' has embedded the SES 74 and the LRD 75 as indicated in the figure.

Likewise the UI 20 could in another obvious variant of the embodiment be integrated in one system thereby having the lighting system 1 as one single unit.

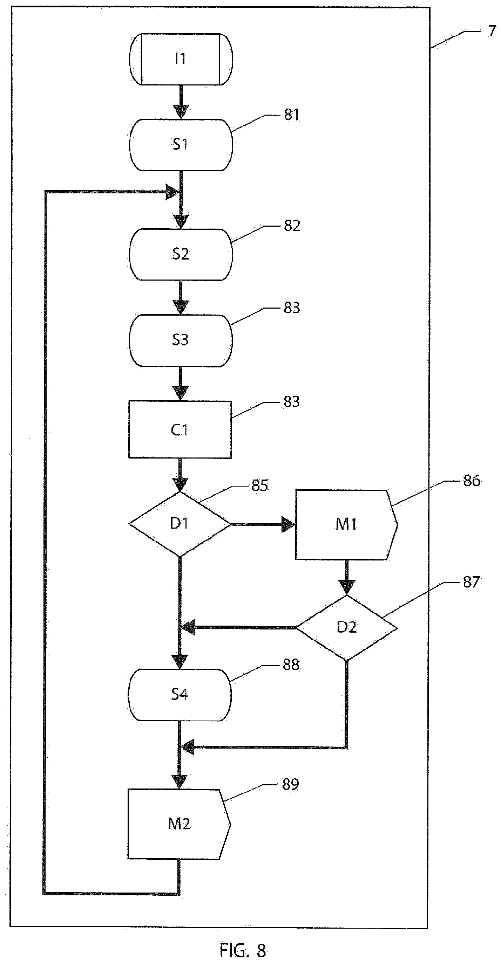

Figure 8 shows a flow diagram of one embodiment of organised memory or sections of the computational means 7 for performing the steps and interactions of retrieving data and information from the sensors and detectors and generating appropriate outputs to be stored for usage by the output units.

Performing an initialisation (II) 80 of the system followed by performing a step that collects user profile data (S I) 81 such as age, sex, chronotype, etc.

The SI 81 is followed by performing a step that collects personal user preferences (S2) 82 such as PC-light, reading light, sealing light, etc.

The S2 82 is followed by performing a step that collects environment parameters (S3) 83 such as light conditions such as background light 8, irradiated light 12 and combinations there of, time of the day as well as data stored in databases or memories such as a storage S4 88 either in or outside the computational unit 2 or the computational means 7.

The steps of collecting data or information S I, S2, S3 81, 82, 83 are followed by a processing step, the computation CI 84, that compute parameters based on data collected in S I, S2, S3, S4 81, 82, 83, 84.

The computation C I 84 is followed by a decision DI step, that decide if the parameters calculated in CI require personal user 70 acknowledgement. If personal user acknowledgement is required, then a manual MI prompt for personal accept is awaited and followed by a decision D2 87 weather the personal user did accept.

If no personal user acknowledgment is required, then the process goes to a step of storage S4 88, were the parameters are stored.

If the personal user accepted in D2 87, then the storage S4 88 is adjusted accordingly.

If the personal user accept in D2 87 is not accepted, then the process is taken to a step of performing and output M2 89 of operational parameters.

The process is then taken to a stage between S I 81 and S2 82 for an iterative loop process iterated from S2 82.

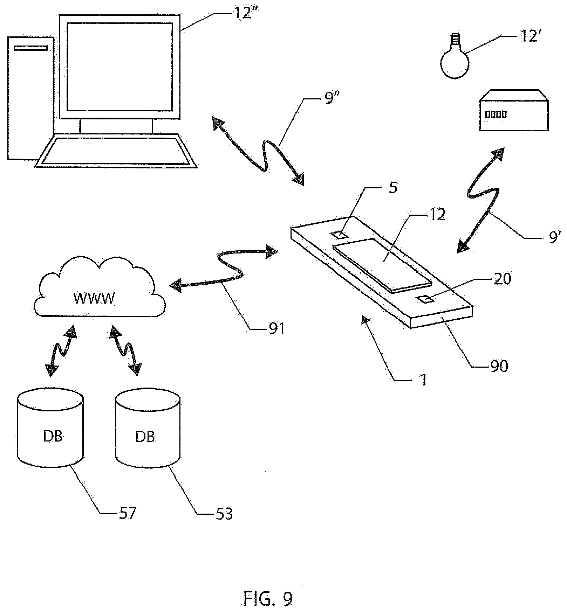

Figure 9 shows an embodiment of a lighting system 1 where the lighting system 1 includes at least a hand held device 90 with a user interface 20 and a light source 12 and a light sensor 5. In one embodiment the user-interface 20 and the light source 12 is the same device and a touch-screen.

The hand held device 90 has a computational unit 2 with computational means 7 embedded along with a wireless communication system for making wireless connections 9', 9" for units with external light sources 12', 12".

In this embodiment the hand held device 90 controls a light source 12' being a lamp, with Light Emitting Diodes (LED) as emitters placed for lighting a room. In this embodiment the hand held device 90 controls a light source 12" being a screen on a personal computer.

The hand held device 90 further has a wireless internet connection 91 for communication with databases DB.

In this embodiment a database DB 53 with data about the personal users work schedule for input to the computational unit 7 for processing.

In this embodiment further a database DB 57 with data about the personal users health record with information.

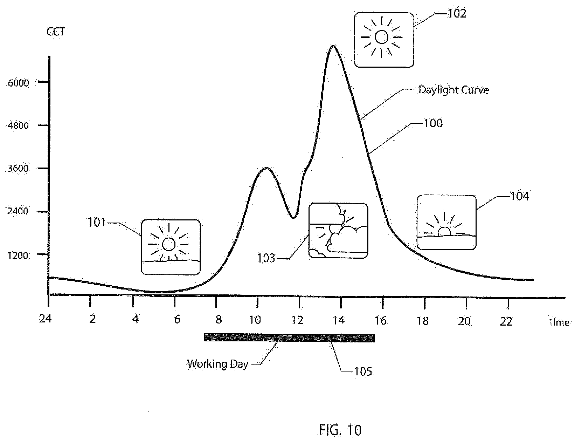

Figure 10 shows a typical daylight curve 100 with the corrected colour temperature (CCT) plotted against time. The daylight curve 100 illustrates variation during a 24- hour period with notable colour temperatures at dawn 101, during clear sky day afternoon 102, on a cloudy day at noon 103 and at dusk 104. Also shown is a typical working day 105 starting at 8:00 finishing at 16:00.

On a different day the daylight curve 100 naturally will emerge different caused by the location of the sun, the weather and the location.

The show daylight curve will be used in the following example as basis or a reference light curve to which the described lighting system will be used to initiate a change of person's circadian state.

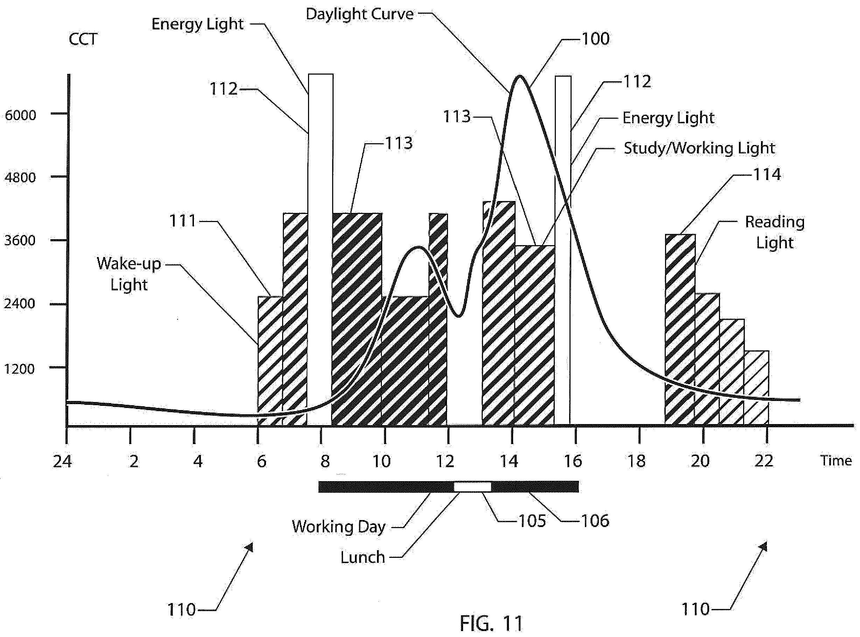

Figure 11 shows the daylight curve 100 from figure 10 with a series of outputs from the light source during the day and as a result of the lighting system 1. The output is series of lights 110 with varying luminosity and chromaticity according to the earlier description such that

06:00-7:30: At dawn the lighting system gradually increases the colour temperature, from warm to cold, of the light source during a period with wake-up light 1 1 1 to help the person to wake up. The wake-up light 111 comes from a light source being a lamp in the bedroom and is controlled by a wireless connection between the computational unit and

7:30-8:30: Just before work, a burst of energy-light 112, primarily blue or cold light at a CCT at about 6,500 K, is emitted to boost the persons energy levels to become alert, effective and productive entering work.

8:30-12:00: During part of a working day 105 a variable study or working light 1 13 is emitted with a CCT varying between approximately 2,400 K and 4,500 K based on the measuring the CCT from the background light with the light sensor and calculating a compensating output via a light source to generate the study or working light 1 13 needed to compensate the background light for a desirable light or a fixed length preselected by the user.

12:00-13 :00: During a lunch break 116, the lighting system is off.

13 :00-15:00: During part of a working day 105 a variable study or working light 113 is emitted using the same principle as between 8:30 - 12.00.

15 :00-15 :30: At the end of the working day 105, a burst of energy-light 112, is emitted to energize the personal user before getting in the car for a drive, thereby minimising the risk of a fatigue that can or will result in an accident.

15:30-18:30: During afternoon and early evening the lighting system is simply off.

18:30-22:00: At the end of the day indoor activities are performed and the lighting system provides a light, which CCT gradually decent from about 4.000 K to 1.500 K to prepare the personal user to enter sleep.

Otherwise, that is during night, the lighting system is in this example idling.

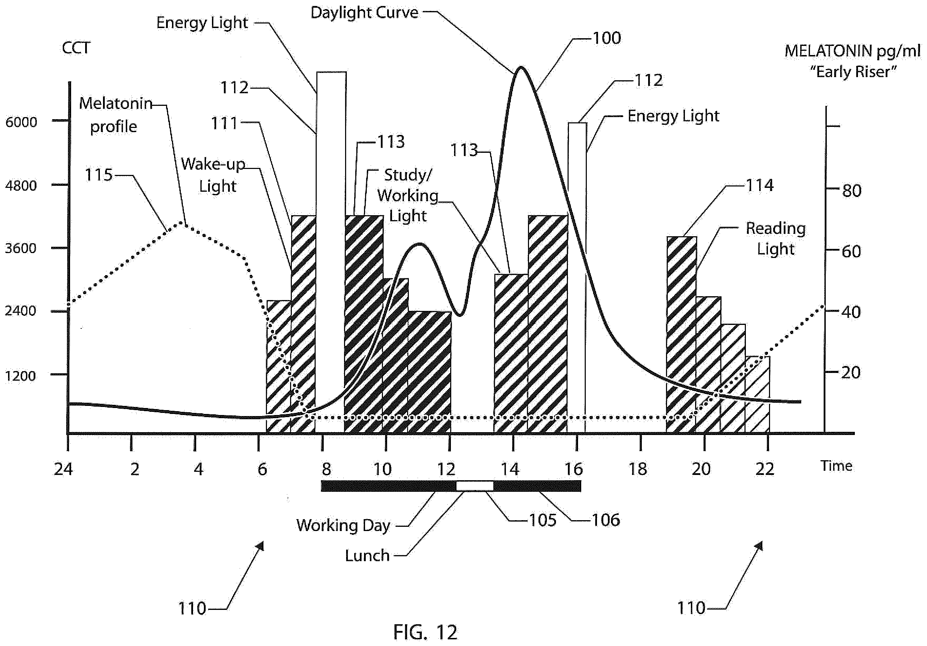

Figure 12 illustrates an example where the personal user is a chronotype A-person, lark, early riser or morning person.

The personal user's melatonin curve has been established and made available to the lighting system. The melatonin profile 115 is shown in the diagram and is shown to peak somewhere between 2:00 and 4:00 decreasing to a minimum around the time of awakening at 7:00.

06:30-07:45: The personal user is wakened by a stimulated spring sunrise profile that is made available by compensating the actual January sunrise. This is done by measuring the background light with the light sensor and comparing this to a stored profile of a spring sunrise and calculating needed values for output in terms of light intensity and CCT. These correction values are used to control a lamp in the sleeping room to gradually increase the light intensity and CCT as shown by the wake-up light 1 1 1 in the figure and to peak at a preset wake-up time just before 8:00.

07:45-08:45: The personal user gets, by way of choice or by preloaded instruction, a boost of energy light 1 12 to fully wake-up and to stop the melatonin production.

08:45-12:00: The personal user is working with a laptop with a screen that is used as a light source and which CCT is controlled by calculated outputs from the computational unit and based on background light, as from the daylight curve 100, characteristics measured by the light sensor.

12:00-13:30: The lighting system is off during and just after the lunch 106.

13 :30-15:45: The personal user is after lunch working in an office with a stationary computer with a screen that is used as a light source and which CCT is controlled by calculated outputs from the computational unit based on the background light, see the daylight curve 100, as measured by the light sensor.

The resulting study or work light 1 13 is seen to increase from a CCT of about 2,500 K to 4,500 K to maintain the personal users energy level for a steady and productive work.

15:45-16: 15: The personal user will be attending a meeting and voluntarily takes a boost of energy light 112 with a CCT of about 6000 K to be energised for the meeting.

16: 15-18:45: The lighting system is off during the meeting and subsequent outdoor activities during the afternoon.

18:45-22:00: The personal user is at home and the light source is the installed lamps. The light sensor measures the background light and the computational means calculates an output that results in a reading light 1 14 with a CCT of about 4000K that will initiate the production of melatonin and decrease gradually to about 1500 K just before preparing bedtime at 22:00 thereby helping the personal user to get the "natural" A- person rhythm and a rhythm that is in sync with a given working day and thereby ex- tending the performance of the personal user and optimising the personal users output.

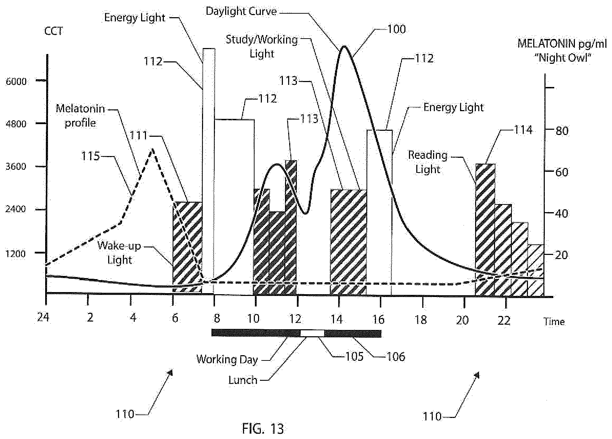

Figure 13 illustrates an example on the same day and with the same work schedule and daily programme as well as physical settings with rooms and computers as in the example with the A-person illustrated in figure 12. However in this example the personal user is a B-person or a night owl or evening person.

Again the personal user's melatonin profile 1 15 is provided in the lighting system. It is seen that the personal user's melatonin profile peaks at around 5:00, which is later than for the A-person type in the example in figure 12.

06:00-07:45: A more steady and constant wake-up light 111 with a CCT of about 2,500 K is provided to adjust the melatonin level, but at the same time allow the personal user to get the required sleep. The personal user is woken at 7:45, were the light source is the lamp in the room is controlled via the computational unit to light with bluish or cold energy light 112 with a CCT of around 6,500 K to abruptly stop the melatonin production and to significantly lower the melatonin level.

07:45-10:00: Likewise, when the personal user is at work the laptop with the screen being the light source gives a steady and relatively cold and bluish energy light 112 with a CCT of around 5000 K for a period to bring the melatonin level to its minimum and to energise the personal user.

10:00-12:00: This is followed by a period of controlled light emission form the laptop screen, where the study or working light 113 varies according to the background light determined by the daylight curve 100.

12:00-13 :30: The lighting system is off during and just after the lunch 106.

13 :30-15:00: During this period the personal user is working on computer which screen emits a study or working light 1 12 with a CCT of about 3,000 K to adjust the background light.

15:30-16:30: The personal user will be attending a meeting and voluntarily takes a boost of energy light 112 with a CCT of about 4,700 K, which is lower than for the A- person not get "too" energised and thereby unnaturally extend the day.

16:30-18:45: The lighting system is off during outdoor activities during the afternoon.

18:45-20:30: The lighting system gives a null-output in the evening since the background light does not need to be adjusted.

20:30-24:00: The reading light 114 is on and adjusts the background light to prepare the personal user for sleep and to adjust the CCT to initiate the production of mela- tonin.

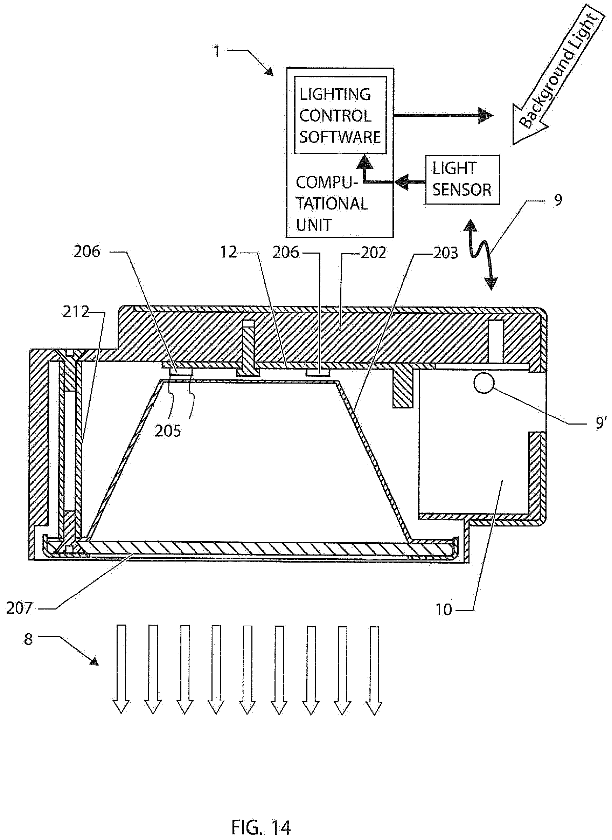

Figure 14 shows a lamp 201 with a housing 202 holding a reflector 203, a light source 12 with a plurality of light emitters 205 here Light Emitting Diodes (LED) that are here arranged in a group of LEDs 206, and a diffuser 207 all configured to emit light 8.

Furthermore, the housing 202 is configured to hold a light control unit 10 with a connector 9' for communicating with a light control unit 10 (only shown schematically in this figure).

In this embodiment there is a spacer 212 for adjusting the distance of the diffuser 207 to the light source 12.

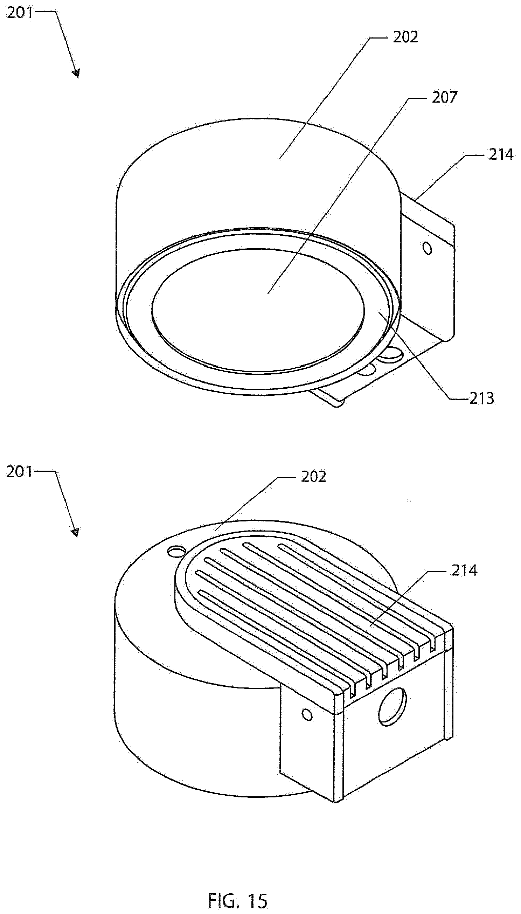

Figure 15 shows an embodiment of a lamp 201 in two different perspective views. The housing 202 is shown with the diffuser 207 sealed by a cover 213 that ensures that ensures that no "false" light escapes. In a particular embodiment the cover 213 also supports the diffuser 207 and the position is controlled by the spacer 212 (not seen in this figure). Furthermore a cooling element 214 is seen.

As such it is seen that the appearance of the lamp 201 is as any other conventional lamps. In this embodiment with side mounts.

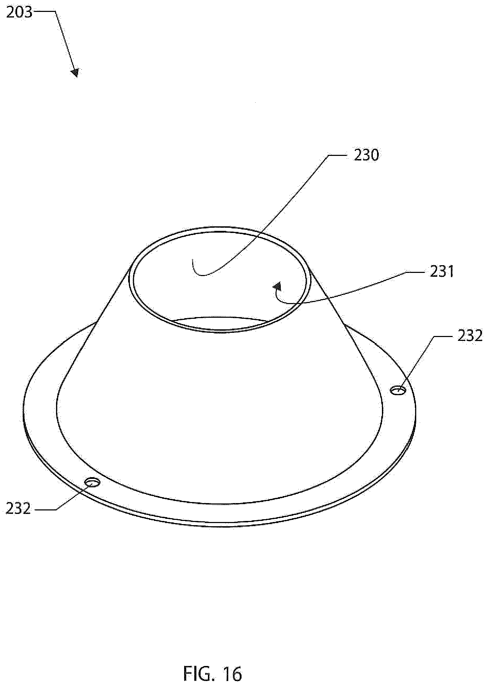

Figure 16 shows a perspective view of an embodiment of a reflector 203. The reflector has a reflector side 230. In this embodiment the reflector side 230 is coated with a reflector coating 231. The reflector coating is in this embodiment a barium sulphate based coating with a so called avian white characteristic.

The reflector 203 has mounting means 232 for mounting the reflector 203 to the housing 202.

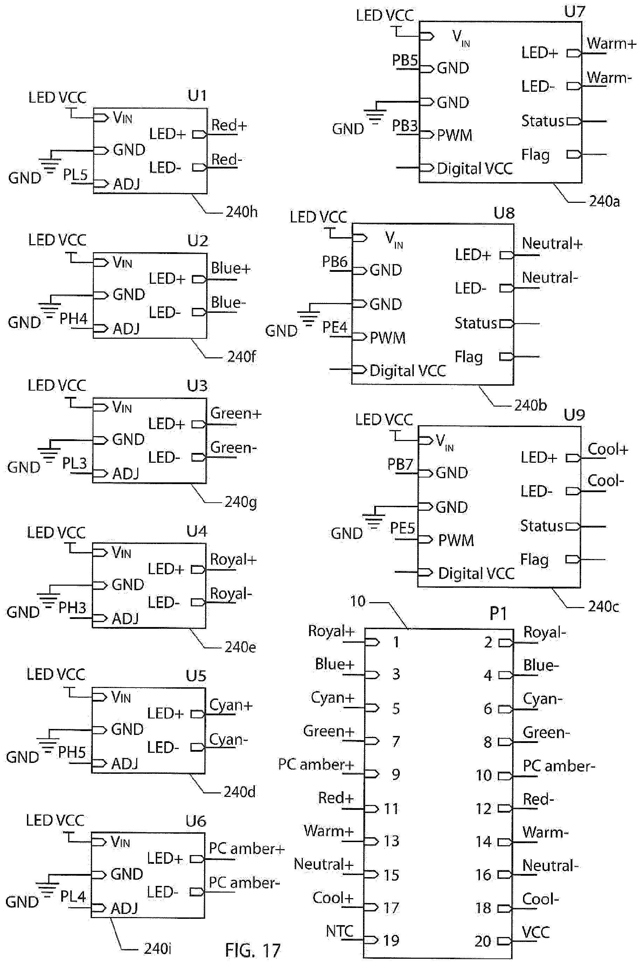

Figure 17 shows light emitter drivers 240 for light emitters 205 or a plurality of coupled light emitters 205.

The light emitter drivers 240 are controlled by a light control unit 10.

In the particular embodiment shown there is:

- a light emitter driver 240a for driving at least one Warm White light emitting diode 205a; such as the LED from Cree Inc. with reference XP-E bin: XPEHEW-H1-8A2- G3-G-01 having a CCT between 2600-3700 K.

- a light emitter driver 240b for driving at least one Neutral White light emitting diode 5b; such as the LED from Cree Inc. with reference XP-E bin: XPEHEW-L1-5C2-Q5- G-01 having a CCT between 3700-5000 K.

- a light emitter driver 240c for driving at least one Cold White light emitting diode 205c; such as the LED from Cree Inc. with reference XP-E bin: XPEHEW-L1-2B0- R4-F-01 having a CCT between 5000-10000 K.

- a light emitter driver 240d for driving at least one Cyan light emitting diode 205d; such as the LED from Phillips Inc. with reference Rebel bin: LXML-PE01-0070 having a CCT between DWL between 490-520 nm.

- a light emitter driver 240e for driving at least one Blue 460nm light emitting diode 205e; such as the LED from Cree Inc. with reference XP-E Bin: XPEBLU-L 1-0000- 00Y02 having a DWL between 450-465 nm.

- a light emitter driver 240f for driving at least one Blue 470nm light emitting diode 205f; such as the LED from Cree Inc. with reference XP-E Bin: XPEROY-L 1-0000- 00A01 having a DWL between 465-485nm.

- a light emitter driver 240g for driving at least one Green light emitting diode 205g; such as the LED from Cree Inc. with reference XP-E Bin: XPEGRN-L1-0000-00B02 having a DWL between 520-535 nm.

- a light emitter driver 240h for driving at least one Red light emitting diode 205h; such as the LED from Cree Inc. with reference XP-E Bin: XPERED-L1 -0000-00301 having a DWL between 620-630 nm.

- a light emitter driver 240i for driving at least one Amber light emitting diode 205i; such as the LED from Phillips Inc. with reference Rebel bin: LXM2PL01-0090-CT1 having a DWL between 588-592 nm.

The light emitting diodes 205a, ... , 205i are not shown, but are implied by the drivers. Likewise the connections between the light control unit 10 and the drivers are not shown.

A person skilled in the art will take this set-up as well as the indicated wavelength ranges as a starting point and by simple experimentation be able by adjustment and slight variations in wavelength be able to achieve the desired result without using the exact same manufacturer or model.

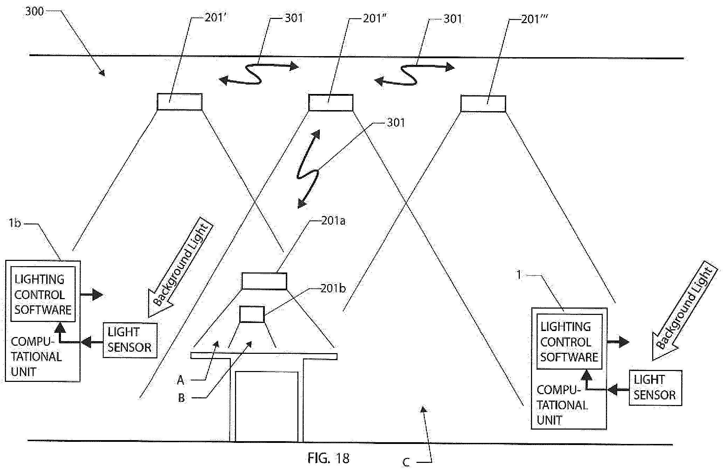

Figure 18 shows a lamp network 300 of a plurality of lamps 201. Each lamp 201 has a connection to a wireless network 301 that can be a standard wireless network with a standard protocol and/or frequency or a private wireless network with a proprietary protocol and/or frequency configured to communicate between a lighting system 1.

The figure exemplifies a lamp network 300 thee lamps 200', 201 ", 20''' placed in a ceiling, a table hung lamp 201a, and a desk lamp 201b, each lamp according to the disclosure. The five lamps illuminate an area dividable into zones. In this case the area is divided into three zones A, B, and C.

In this particular embodiment a lighting system 1 controls zone C by primarily regulating lamp 201''', to a less degree lamp 201" and to an even lesser degree lamp 201'.

In this particular embodiment a lighting system lb controls zone B by primarily regulating lamp 201b, to a less degree lamp 201a and to an even lesser degree lamps 201', 201", and 201'''.

In this particular embodiment the zone A is left uncontrolled, but naturally dependent on the control of zone B and C.

This embodiment is only an example and a person skilled in the art will only find it natural to explore other configurations according to the area at hand.

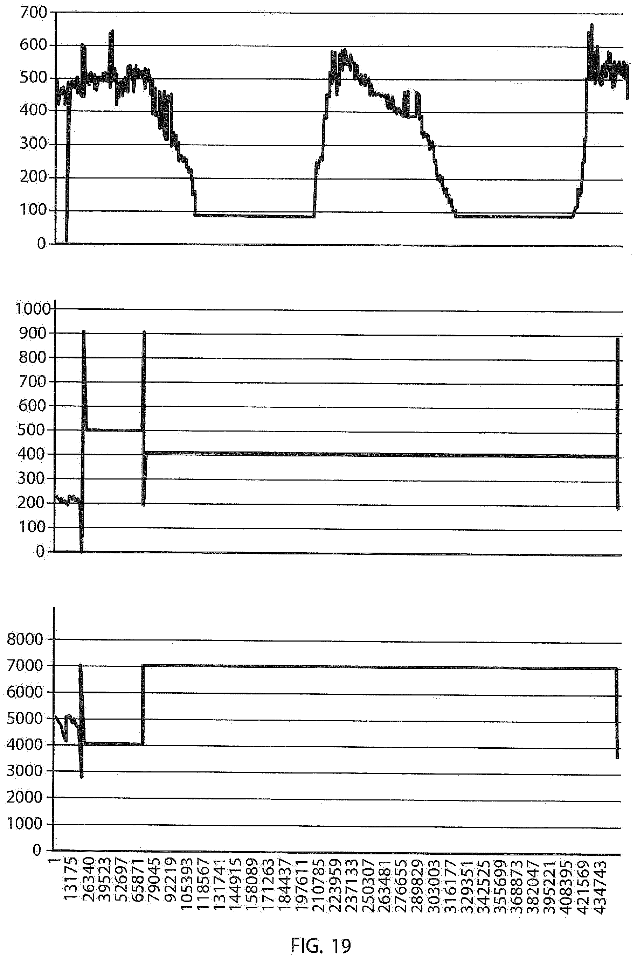

Finally, figure 19 shows a subset of data from an embodiment of the disclosure as collected, processed and outputted for a female, who has pre-selected a cronotype suitable for a an evening person.

The top curve shows a scaled (1-1024) measure of light intensity as measured by a light sensor in an area or zone similar to zone B from figure 18 and in a room with windows. A lamp according to an embodiment of the disclosure was placed similar to 201b in figure 18.

The time series covers a time period from a Friday morning to a Sunday morning and the variation in the sunlight over the days is clearly observed. Spikes in the data over the period come from turning on lights in the room and the lamp according to the disclosure. The middle curve shows the light intensity from the lamp as regulated by the lighting system automatically during the Friday morning and manually (boosts) shown as spikes. From Friday afternoon and through the weekend, the lamp was left on at a fixed intensity as also showing as a constant floor in the top curve after sunset.

For a short period Friday morning, the system was turned off.

The bottom curve shows the colour temperature of the lamp as regulated by the lighting system.

It is observed that the system is configured to be able to regulate intensity as well as colour temperature continuously. The two small declines in the colour temperature and the corresponding decline in intensity is to maintain a substantially constant light intensity and colour temperature in the working area around the lamp.

A first peak in intentity and increase in colour temperature to about 7000 K is seen and caused by a request by the user to get a boost by a quantum of "intense blue light".

For the remaining period over the weekend, the lamp is switched on at a constant intensity/colour temperature and working a safety light with low energy consumption.

The above description can be summarised in the following points:

1. A lighting system (1) for initiating change of a mammal's (70) circadian state or well-being state, comprising:

- at least one computational unit (2) with a memory, a processor, at least one input (3) and at least one output (4)
- at least one light sensor (5) with spectral and luminosity sensitivity and means for providing information through a connection (6) to said least one input (3) of the computational unit (2),

which computational unit (2) has computational means (7) that are arranged for collecting, storing, and processing data from at least the light sensor (5) making operational information (77) and comparing said operational information (77) with state information (76) about the mammal's (70) circadian state or well-being state and which computational means (7) compares the operational information (77) and the state in- formation (76) for activating means for generating at least one output signal (4)at least one connector (9) for communicating with and controlling at least one light source (12,201) and which

- computational unit (2) is configured to calculate and least one output signal (4) configured to output the setting of at least one light source (12) or lamp (201) to

- a colour temperature range between 2700 - 7000 K;

- an intensity range between 10 to 1000 lumens; and
- a Ra-index greater than 92 at all values of colour temperature and intensity.

2. A lighting system (1) according to point 1, characterised in that, said lighting system (1) further comprises:

- at least one user interface (20) with a connection to of the computational unit (2) for making personal data (21) available to the computational means (7).

3. A lighting system (1) according to any of points 1 to 2, characterised in that, said lighting system (1) further comprises:

- at least one computational interface for connecting to at least one second computational unit for retrieving and storing information.

4. A lighting system (1) according to any of points 1 to 3, characterised in that, said lighting system (1) further comprises:

- at least one biometrical sensor unit (31) with a connection to the computational unit (2) for providing biometric data (30) to the computational means (7).

5. A lighting system (1) according to any of points 1 to 4, characterised in that, said lighting system (1) further comprises:

- at least one activity sensor unit (41) for providing activity data (40) to the computational means (7).

6. A lighting system (1) according to any of points 1 to 5, characterised in that, said lighting system (1) to the computational unit (2) via a connection further comprises at least one additional sensor or detector unit being a unit such as:

- a time detector or unit (51),
- a location detector or unit (52),
- a work schedule detector or unit (53),
- a sleep pattern detector or unit (54),
- a daylight detector or unit (55),
- a mood detector or unit (56),
- a health detector or unit (57),
- a date detector or unit (58),
- an mammal energy detector or unit (59), or
- a connection detector or unit for receiving data or information from an unlisted unit via a protocol.

7. A lighting system (1) according to any of the points 1 to 6, characterised in that, said units or detectors (5, 20, 31, 41, 51, 52, 53, 54, 55, 56, 57, 58, 59) are connected to the computational unit (2) or said units or detectors (5, 20, 31, 41, 51, 52, 53, 54, 55, 56, 57, 58, 59) are embedded in the computational unit (2).

8. A lamp (12,201) emitting light (8), comprising:

- a housing (202) configured to hold a
- a reflector (203) configured to reflect and/or direct light from
- a least one light source (12) through
- a diffuser (207)

wherein said lamp (201) further comprises a light control unit (10) with at least one connector (9') for communicating with and receiving regulating information from a lighting system (1) according to any of points 1 to 7 and configured to vary at least one light source (12) so the light (8) emitted from the lamp (201) has

- a colour temperature range between 2700 - 7000 K;
- an intensity range between 10 to 1000 lumens; and
- a Ra-index greater than 92 at all values of colour temperature and intensity.

9. A lamp (201) according to point 8, characterised in that, the light source (12) comprises a plurality of light emitters (205), preferably light emitting diodes or LEDs.

10. A lamp (1) according to any of points 8 to 9, characterised in that, light emitters (205) in the light source (12) are configured on the basis colour mixing of chromaticity binning using a plurality of bins, individually or pre-selected plurality of bins formed to generate a warm white, neutral white, or cool white or combinations thereof.

11. A lamp (12) according to any of points 8 to 10, characterised in that, that the light source (12):

-   comprises at least one red, at least one blue, at least one green, at least one Royal blue, at least one cyan, at least amber light emitters in combination with at least a white light emitter; or
-   comprises at least one red, at least one green, and at least blue light emitters, or comprises at least one white/yellow and phosphor converted light emitters in combination with at least one blue, and at least a first and a second red light emitter combination with at least a white light emitter;

where said least one white light emitter comprises

-   at least one emitting warm white light emitting diode having a CCT between 2600 K - 3700K, at least one emitting neutral white light emitting diode having a CCT between 3700 K - 5000 K, and at least one emitting cold white light emitting diode having a CCT between 5000 K - 10000 K; or
-   at least one white light emitter has a CCT between 2700 K or 7000 K.

12. A lamp (201) according to point 11, characterised in that, the light control unit (10) is configured to vary each light emitter (205) by means of a

-   a lookup table,
-   programmed functions, or
-   an interpolation of said lookup table or programmed functions, or both.

13. A lamp (201) according to any of points 8 to 12, characterised in that, the lamp (201) further includes at least a second connector for communicating with receiving regulating information via a standard wireless network (301) with a standard protocol such as CAN, a private wireless lamp network (301) for communicating with at least one other lamp ( ), or a wired network with a standard protocol such as CAN.

14. A kit of at least one lamp (201) according to any of points 8-13 and at least one lighting system (1) according to any of points 1-7.

15. A network of lamps (300) comprising a plurality of lamps (201) according to any of points 1-6 and at least one lighting system (1) according to any of point 1-X, characterised in that, said lamps (201) and least one lighting system (1) are configured in zones (A, B, ... ), each zone (A, B, ...) being controlled or regulated by at least one lamp (201) and at least one lighting system (1).

16. A method for initiating change of a mammal's (70) circadian state or well-being state using at least one lamp (201) according to any of points 8-13 and at least one lighting system (1) according to any of points 1-7, the method comprising:

-   measuring the intensity and the chromaticity of light surrounding (71, 8, 13) the mammal (70) and providing the measuring as light data (83)
-   collecting, storing, and processing measured light data and making operational information (77) thereof
-   generating an output signal (4) for initiating change of the mammals (70) surroundings.

17. A method according to point 16 further comprising:

-   retrieving or storing data about a mammals (70) circadian state or wel- being state and making state information (76) thereof
-   comparing the operational information (77) and the state information (76) before generating an output (4) based on the comparison.

18. A method according to point 16 or 17 further comprising:

- varying at least one light source's (12) luminosity or chromaticity, which least one light source (12) is present in the surroundings (71) of the mammal (70) thereby generating an output signal (4) for initiating a change of the mammals (70) surrounding (71) light conditions (13).

PARTIAL ITEM LIST

| | |
|---|---|
| 1000 | lighting system |
| 1002 | time detector or unit |
| 1004 | user interface |
| 1006 | computational unit |
| 1008 | lamp |
| 1010 | current time |
| 1012 | type parameter |
| 1014 | gender parameter |
| 1016 | age parameter |
| 1018 | first function |
| 1020 | second function |
| 1022 | output control parameter |
| 1024 | first peak |
| 1026 | first maximum |
| 1027 | first time of the day |
| 1028 | first full width at half maximum |
| 1030 | second peak |
| 1032 | second maximum |
| 1033 | second time of the day |
| 1034 | second full width at half maximum |
| 1035 | first base function $T_0(t)$ |
| 1036 | first type of person male |
| 1038 | first type of person female |
| 1040 | second type of person male |
| 1042 | second type of person female |
| 1044 | first range of local time |
| 1045 | colour temperature base level |
| 1046 | first depression |
| 1048 | first minimum |
| 1049 | third time of the day |
| 1050 | fifth peak |
| 1052 | fifth maximum |
| 1053 | seventh time of the day |
| 1054 | fifth full width at half maximum |
| 1056 | third peak |
| 1058 | third maximum |
| 1059 | fourth time of the day |
| 1060 | third full width at half maximum |
| 1062 | fourth peak |
| 1064 | fourth maximum |
| 1065 | fifth time of the day |
| 1066 | fourth full width at half maximum |
| 1068 | second depression |
| 1070 | second minimum |
| 1071 | sixth time of the day |
| 1072 | fourth base function $I_0(t)$ |
| 1074 | 30 year old |
| 1076 | 60 year old |

1078    intensity base level
1080    second and third range of local time
1082    fifth base function $c_{age}(z)$

**Claims**

1.  A non-therapeutical method for providing an output control parameter associated with a specific person for controlling a light output of a lamp adapted to vary said light output by said output control parameter, said light output being **characterised by** a colour temperature and said control parameter comprising a colour temperature control parameter for controlling the colour temperature of said light output, said method comprising:

    providing a current time parameter representing the current local time of the day,
    providing a type parameter from a range of type parameters comprising at least a parameter representing a first person type and a parameter representing a second person type,
    providing a gender parameter from a range of gender parameters comprising a parameter representing a male person and a parameter representing a female person, and
    **in a first alternative** providing a first function being a multivariable function and comprising:

    a first variable representing the local time, a second variable representing said range of type parameters, and a third variable representing said range of gender parameters as input,
    a range of colour temperature control parameters as output;
    said first function defining a first peak and a second peak of said range of colour temperature control parameters with respect to said first variable,
    said first peak having a first maximum at a first time of the day and a first full width at half maximum of at least 30 minutes,
    said second peak having a second maximum at a second time of the day that is after said first time of the day and a second full width at half maximum of at least 30 minutes,
    said first function providing at a fixed value of said third variable an output representing a lower colour temperature at said first time of the day for said first person type than for said second person type and an output representing a higher colour temperature at said second time of the day for said first person type than for said second person type, and
    said first function further providing at a fixed value of said second variable an output representing a lower colour temperature at said first time of the day and said second time of the day for a male person than for a female person,

    or **in a second alternative** providing a first function being a multivariable function representing:

    $$T(t,\ x,\ y) = (T_0(t) - T_b) \cdot c_{type}(x) \cdot c_{gender}(y) + T_b,$$

    where $t$ is a first variable representing the local time, x is a second variable representing said range of type parameters, and $y$ is a third variable representing said range of gender parameters, and said first, second and third variables constitutes an input for said first function,
    $T(t, x, y)$ is the output of said first function and covers a range of temperature control parameters,
    $T_0(t)$ is a first base function of the first variable $t$ and defining a first peak and a second peak of said range of colour temperature control,
    said first peak having a first maximum at a first time of the day and a first full width at half maximum of at least 30 minutes,
    said second peak having a second maximum at a second time of the day that is after said first time of the day and a second full width at half maximum of at least 30 minutes,
    $c_{type}(x)$ is a second base function of the second variable $x$ and providing an output corresponding to a lower colour temperature at said first time of the day for said first person type than for said second person type and an output representing a higher colour temperature at said second time of the day for said first person type than for said second person type,
    $c_{gender}(y)$ is a third base function of the third variable $y$ and providing an output corresponding to a lower colour temperature at said first time of the day and said second time of the day for a male person than for a female person, and

$T_b$ is a colour temperature base level that is a positive constant for all values of said first, second, and third variables.

and **in both the first and the second alternatives** inputting into said first function said current time parameter as said first variable, said type parameter for said specific person as said second variable, and said gender parameter for said specific person as said third variable to yield said colour temperature control parameter as the output from said first function.

2. The method according to claim 1 **in the first alternative characterised by** said first function defining a colour temperature base level representing a positive constant colour temperature over a first range of the local time, and said colour temperature being independent of said second variable and said third variable over said first range, and said first full width at half maximum representing the width of said first peak at a colour temperature corresponding to the mean of the colour temperature of said first maximum and the colour temperature at said colour temperature base level, and said second full width at half maximum representing the width of said second peak at a colour temperature corresponding to the mean of the colour temperature of said second maximum and the colour temperature at said colour temperature base level, or In **the second alternative characterised by** said first base function $T_0(t)$ being zero or approximately zero over a first range of the local time and said first full width at half maximum representing the width of said first peak at the middle between said first maximum and $T_b$, and said second full width at half maximum representing the width of said second peak at the middle between said second maximum and $T_b$.

3. The method according to any of the claims 1-2 **in the first alternative characterised by** said first function defining a first depression between said first peak and said second peak at fixed values of said second and third variables, said first depression having a first minimum at a third time of the day and said first function providing an output at said third time of the day that is the same or approximately the same for all values of said second variable and said third variable, or **in the second alternative characterised by** said first base function $T_0(t)$ defining a first depression between said first peak and said second peak, said first depression having a first minimum at a third time of the day, said second base function $c_{type}(x)$ providing an output at said third time of the day that is the same or approximately the same for all values of said second variable $x$, and said third base function $c_{type}(x)$ providing an output at said third time of the day that is the same or approximately the same for all values of said third variable $y$.

4. The method according to any of the claims 1-3, **characterised by** said first time of the day being before noon according to the local time and/or said second time of the day being after noon according to the local time.

5. The method according to any of the claims 1-4, said light output further being **characterised by** an intensity and said control parameter further comprising an intensity control parameter for controlling the intensity of said light output, and said method further comprising:

**in the first alternative** providing a second function comprising:

a fourth variable representing the local time,
a range of intensity control parameters as output,
said second function defining a third peak and a fourth peak of said range of intensity control parameters,
said third peak having a third maximum at a fourth time of day and a third full width at half maximum of at least 30 minutes, and
said fourth peak having a fourth maximum at a fifth time of the day that is after said fourth time of the day and a fourth full width at half maximum of at least 30 minutes,

or **in the second alternative** providing a second function representing:

$$I(t) = I_0(t) \cdot c,$$

where $t$ is a fourth variable representing the local time and said fourth variable constitutes an input for said second function,
$I(t)$ is the output covering a range of intensity control parameters;,
$I_0(t)$ is a fourth base function of the fourth variable $t$ and defining a third peak and a fourth peak of said range of colour temperature control,
said third peak having a third maximum at a fourth time of day and a third full width at half maximum of at least 30 minutes,
said second peak having a fourth maximum at a fifth time of the day that is after said fourth time of the day and a fourth full width at half maximum of at least 30 minutes, and
c is a correction factor,

and in **both the first and the second alternatives** inputting into said second function said current time parameter as said fourth variable to yield said intensity control parameter as the output from said second function.

6. The method according to claim 5, **in the first alternative characterised by** said second function defining an intensity base level representing a positive constant intensity over a second range of the local time, and said third full width at half maximum representing the width of said third peak at an intensity corresponding to the mean of the intensity of said third maximum and the intensity at said intensity base level, and/or said fourth full width at half maximum representing the width of said fourth peak at an intensity corresponding to the mean of the intensity of said fourth maximum and the intensity at said intensity base level, or **in the second alternative characterised by** said fourth base function $I_0(t)$ being equal to or approximately equal to an intensity base level over a second range of the local time where said intensity base level is a constant; said third full width at half maximum representing the width of said third peak at the middle between said third maximum and said intensity base level, and/or said fourth full width at half maximum representing the width of said fourth peak at the middle between said fourth maximum and said intensity base level.

7. The method according to any of the claims 5-6, **in the first alternative characterised by** said second function defining a second depression between said third peak and said fourth peak, said second depression having a second minimum at a sixth time of the day, or **in the second alternative characterised by** said fourth base function $I_0(t)$ defining a second depression between said third peak and said fourth peak, said second depression having a first minimum at a sixth time of the day.

8. The method according to any of the claims 5-7, **characterised by** said first time of the day and said fourth time of the day being the same or approximately the same time of the day, and/or said second time of the day and said fifth time of the day being the same or approximately the same time of the day, and/or said third time of the day and said sixth time of the day being the same or approximately the same time of the day.

9. The method according to any of the claims 5-8, **characterised by** said method further comprising:

   providing an age parameter from a range of age parameters representing different ages of a person, and
   **in the first alternative** said second function further being a multivariable function and further comprising:

      a fifth variable representing said range of age parameters as input, and
      said second function providing at a fixed value of said fourth variable an output that increases monotonically with said fifth variable,

   or **in the second alternative** said second function further being a multivariable function representing

$$I(t,\ z) = I_0(t) \cdot c_{age}(z),$$

   where $t$ is said fourth variable representing the local time, and $z$ is a fifth variable representing said range of age parameters, and said fourth and fifth variables constitute said input for said second function,
   $I(t,\ z)$ is the output of said second function and covers said range of intensity control parameters,
   $I_0(t)$ is said fourth base function of the fourth variable $t$, and
   $c_{age}(z)$ is a fifth base function of said fifth variable $z$, said fifth base function constituting said correction factor $c$ and providing an output representing an intensity that increases monotonically with the age of said person, and **in both the first and the second alternatives** said method further comprising:

      inputting into said second function said age parameter for said specific person as said fifth variable in addition to said current time parameter as said fourth variable to yield said intensity control parameter as the output from said second function.

10. The method according to claim 9 **In the first alternative characterised by** said second function providing at a fixed value of said fourth variable an output that increases exponentially with said fifth variable, or **in the second alternative characterised by** said fifth base function $c_{age}(z)$ representing:

$$c_{age}(z) = 1/(2\wedge(13/(z-25))),$$

where the parameter $z$ is given in the unit of years.

11. The method according to any of the claims 5-10, **in the first alternative characterised by** said second function being constant or approximately constant over a third range of the local time at a fixed value of said fifth variable, and said first function defining a fifth peak having a fifth maximum at a seventh time of day and a fifth full width at half maximum of at least 15 minutes, and said third range of the local time being after said second and/or said fourth time of the day and comprising said seventh time of the day, or **in the second alternative characterised by** said second base function being constant or approximately constant over a third range of the local time and said first base function defining a fifth peak having a fifth maximum at a seventh time of day and a fifth full width at half maximum of at least 15 minutes, and said third range of the local time being after said second time of the day and comprising said seventh time of the day.

12. A non-therapeutical method for providing light for a specific person, said method comprising

    providing a lamp adapted to vary its light output by an output control parameter, and
    providing said output control parameter by the non-therapeutical method according to any of the claims 1 to 11.

13. Lighting system (1) adapted to provide an output control parameter associated with a specific person for controlling a light output of a lamp adapted to vary said light output by said output control parameter, said light output being **characterised by** a colour temperature and said control parameter comprising a colour temperature control parameter for controlling the colour temperature of said light output, said lighting system comprising:

    a computational unit (2) adapted to provide said output and adapted to perform the following steps:

        providing a current time parameter representing the current local time of the day,
        providing a type parameter from a range of type parameters comprising at least a parameter representing a first person type and a parameter representing a second person type,
        providing a gender parameter from a range of gender parameters comprising a parameter representing a male person and a parameter representing a female person, and
        **in a first alternative** providing a first function being a multivariable function and comprising:

            a first variable representing the local time, a second variable representing said range of type parameters, and a third variable representing said range of gender parameters as input,
            a range of colour temperature control parameters as output;
            said first function defining a first peak and a second peak of said range of colour temperature control parameters with respect to said first variable,
            said first peak having a first maximum at a first time of the day and a first full width at half maximum of at least 30 minutes,
            said second peak having a second maximum at a second time of the day that is after said first time of the day and a second full width at half maximum of at least 30 minutes,
            said first function providing at a fixed value of said third variable an output representing a lower colour temperature at said first time of the day for said first person type than for said second person type and an output representing a higher colour temperature at said second time of the day for said first person type than for said second person type, and
            said first function further providing at a fixed value of said second variable an output representing a lower colour temperature at said first time of the day and said second time of the day for a male person than for a female person,
            or **in a second alternative** providing a first function being a multivariable function representing:

$$T(t, x, y) = (T_0(t) - T_b) \cdot c_{type}(x) \cdot c_{gender}(y) + T_b,$$

where $t$ is a first variable representing the local time, $x$ is a second variable representing said range of type parameters, and $y$ is a third variable representing said range of gender parameters, and said first, second and third variables constitutes an input for said first function,

$T(t, x, y)$ is the output of said first function and covers a range of temperature control parameters,

$T_0(t)$ is a first base function of the first variable $t$ and defining a first peak and a second peak of said range of colour temperature control,

said first peak having a first maximum at a first time of the day and a first full width at half maximum of at least 30 minutes,

said second peak having a second maximum at a second time of the day that is after said first time of the day and a second full width at half maximum of at least 30 minutes,

$c_{type}(x)$ is a second base function of the second variable x and providing an output corresponding to a lower colour temperature at said first time of the day for said first person type than for said second person type and an output representing a higher colour temperature at said second time of the day for said first person type than for said second person type,

$c_{gender}(y)$ is a third base function of the third variable $y$ and providing an output corresponding to a lower colour temperature at said first time of the day and said second time of the day for a male person than for a female person, and

$T_b$ is a colour temperature base level that is a positive constant for all values of said first, second, and third variables,

and **in both the first and the second alternatives** inputting into said first function said current time parameter as said first variable, said type parameter for said specific person as said second variable, and said gender parameter for said specific person as said third variable to yield said colour temperature control parameter as the output from said first function,

said lighting system further comprising:

a time detector or unit (51) connected to said computational unit for providing said local time of the day; and

a user interface (20) connected to said computational unit for providing a type parameter from a range of type parameters comprising at least
a parameter representing a first person type and a parameter representing a second person type, and for providing a gender parameter from a range of gender parameters comprising a parameter representing a male person and a parameter representing a female person.

14. The lighting system according to claim 13, **characterised by** said user interface further being for manually providing an age parameter from a range of age parameters representing different ages of a person.

15. The apparatus according to claim 13, being adapted to perform an implementation of the method according to any of the claims 1-12 for non-therapeutical or therapeutical application.

16. A kit comprising a lamp adapted to vary its light output by an output control parameter and lighting system according to any of the claims 13 to 15 for providing said output control parameter for controlling the light output of said lamp, said lighting system further being connected to said lamp for communicating said output control parameter to said lamp.

**Patentansprüche**

1. Nicht therapeutisches Verfahren zum Bereitstellen eines Ausgabesteuerparameters mit einer spezifischen Person verbunden zum Steuern einer Lichtausgabe einer Lampe, die dazu eingerichtet ist, die Lichtausgabe durch den Ausgabesteuerparameter zu variieren, wobei die Lichtausgabe durch eine Farbtemperatur gekennzeichnet ist, und der Steuerparameter einen Farbtemperatursteuerparameter zum Steuern der Farbtemperatur der Lichtausgabe umfasst, wobei das Verfahren umfasst:

Bereitstellen eines aktuellen Zeitparameters, der die aktuelle lokale Tageszeit repräsentiert,
Bereitstellen eines Typparameters von einem Bereich von Typparametern umfassend mindestens einen Parameter, der einen ersten Personentyp repräsentiert, und einen Parameter, der einen zweiten Personentyp repräsentiert,

Bereitstellen eines Geschlechtsparameters von einem Bereich von Geschlechtsparametern umfassend einen Parameter, der eine männliche Person repräsentiert, und einen Parameter, der eine weibliche Person repräsentiert, und

in **einer ersten Alternative** Bereitstellen einer ersten Funktion, die eine multivariable Funktion ist und umfassend:

eine erste Variable, welche die lokale Zeit repräsentiert, eine zweite Variable, welche den Bereich von Typparametern repräsentiert, und eine dritte Variable, welche den Bereich von Geschlechtsparametern als Eingabe repräsentiert,

einen Bereich von Farbtemperatursteuerparametern als Ausgabe;

wobei die erste Funktion eine erste Spitze und eine zweite Spitze des Bereichs von Farbtemperatursteuerparametern bezüglich der ersten Variable definiert,

wobei die erste Spitze ein erstes Maximum zu einer ersten Tageszeit und eine erste volle Breite bei dem halben Maximum von mindestens 30 Minuten aufweist,

wobei die zweite Spitze ein zweites Maximum zu einer zweiten Tageszeit, die nach der ersten Tageszeit liegt, und eine zweite volle Breite bei dem halben Maximum von mindestens 30 Minuten aufweist,

wobei die erste Funktion zu einem Festwert der dritten Variable eine Ausgabe bereitstellt, die eine niedrigere Farbtemperatur zu der ersten Tageszeit für den ersten Personentyp als für den zweiten Personentyp repräsentiert, und eine Ausgabe, die eine höhere Farbtemperatur zu der zweiten Tageszeit für den ersten Personentyp als für den zweiten Personentyp repräsentiert, und

wobei die erste Funktion ferner zu einem Festwert der zweiten Variable eine Ausgabe bereitstellt, die eine niedrigere Farbtemperatur zu der ersten Tageszeit und der zweiten Tageszeit für eine männliche Person als für eine weibliche Person repräsentiert,

oder **in einer zweiten Alternative** Bereitstellen einer ersten Funktion, die eine multivariable Funktion ist, repräsentierend:

$$T(t,\ x,\ y) = (T_0(t) - T_b) \cdot c_{type}(x) \cdot c_{gender}(y) + T_b,$$

wobei $t$ eine erste Variable ist, welche die lokale Zeit repräsentiert, $x$ eine zweite Variable ist, welche den Bereich von Typparametern repräsentiert, und $y$ eine dritte Variable ist, welche den Bereich von Geschlechtsparametern repräsentiert, und die erste, die zweite und die dritte Variable eine Eingabe für die erste Funktion darstellen,

wobei $T(t,\ x,\ y)$ die Ausgabe der ersten Funktion ist und einen Bereich von Temperatursteuerparametern abdeckt,

wobei $T_0(t)$ eine erste Basisfunktion der ersten Variable $t$ ist und eine erste Spitze und eine zweite Spitze des Bereichs von Farbtemperatursteuerung definiert,

wobei die erste Spitze ein erstes Maximum zu einer ersten Tageszeit und eine erste volle Breite bei dem halben Maximum von mindestens 30 Minuten aufweist,

wobei die zweite Spitze ein zweites Maximum zu einer zweiten Tageszeit, die nach der ersten Tageszeit liegt, und eine zweite volle Breite bei dem halben Maximum von mindestens 30 Minuten aufweist,

wobei $c_{type}(x)$ eine zweite Basisfunktion der zweiten Variable $x$ ist und eine Ausgabe entsprechend einer niedrigeren Farbtemperatur zu der ersten Tageszeit für den ersten Personentyp als für den zweiten Personentyp bereitstellt, und eine Ausgabe, die eine höhere Farbtemperatur zu der zweiten Tageszeit für den ersten Personentyp als für den zweiten Personentyp repräsentiert,

wobei $c_{gender}(y)$ eine dritte Basisfunktion der dritten Variable $y$ ist und eine Ausgabe entsprechend einer niedrigeren Farbtemperatur zu der ersten Tageszeit und der zweiten Tageszeit für eine männliche Person als für eine weibliche Person bereitstellt, und

wobei $T_b$ ein Farbtemperaturbasisniveau ist, das eine positive Konstante für alle Werte der ersten, der zweiten und der dritten Variable ist,

und **in sowohl der ersten als auch der zweiten Alternative** Eingeben des aktuellen Zeitparameters als der ersten Variable, des Typparameters für die spezifische Person der zweiten Variable, und des Geschlechtsparameters für die spezifische Person als der dritten Variable in die erste Funktion, um die Farbtemperatursteuerparameter als die Ausgabe von der ersten Funktion zu geben.

**2.** Verfahren nach Anspruch 1, **in der ersten Alternative dadurch gekennzeichnet, dass** die erste Funktion ein Farbtemperaturbasisniveau definiert, das eine positive konstante Farbtemperatur über einen ersten Bereich der lokalen Zeit repräsentiert, und wobei die Farbtemperatur unabhängig von der zweiten Variable und der dritten Variable über den ersten Bereich ist, und wobei die erste volle Breite bei dem halben Maximum die Breite der ersten Spitze bei einer Farbtemperatur entsprechend dem mittleren Wert der Farbtemperatur des ersten Maximums und der Farbtemperatur bei dem Farbtemperatur-basisniveau repräsentiert, und die zweite volle Breite bei dem halben Maximum die Breite der zweiten Spitze bei einer Farbtemperatur entsprechend dem mittleren Wert der Farbtemperatur des zweiten Maximums und der Farbtemperatur bei dem Farbtemperatur-basisniveau repräsentiert, oder **in der zweiten Alternative dadurch gekennzeichnet, dass** die erste Basisfunktion $T_0(t)$ null oder ungefähr null über einen ersten Bereich der lokalen Zeit ist, und die erste volle Breite bei dem halben Maximum die Breite der ersten Spitze in der Mitte zwischen dem ersten Maximum und $T_b$, epräsentiert, und die zweite volle Breite bei dem halben Maximum die Breite der zweiten Spitze in der Mitte zwischen dem zweiten Maximum und $T_b$ repräsentiert.

**3.** Verfahren nach einem der Ansprüche 1-2, **in der ersten Alternative dadurch gekennzeichnet, dass** die erste Funktion eine erste Vertiefung zwischen der ersten Spitze und der zweiten Spitze zu Festwerten der zweiten der dritten Variable definiert, wobei die erste Vertiefung ein erstes Minimum zu einer dritten Tageszeit aufweist, und die erste Funktion eine Ausgabe zu der dritten Tageszeit bereitstellt, welche die gleiche oder ungefähr die gleiche für alle Werte der zweiten Variable und der dritten Variable ist, oder **in der zweiten Alternative dadurch gekennzeichnet, dass** die erste Basisfunktion $T_0(t)$ eine erste Vertiefung zwischen der ersten Spitze und der zweiten Spitze definiert, wobei die erste Vertiefung ein erstes Minimum zu einer dritten Tageszeit aufweist, die zweite Basisfunktion $c_{type}(x)$ eine Ausgabe zu der dritten Tageszeit bereitstellt, welche die gleiche oder ungefähr die gleiche für alle Werte der zweiten Variable $x$ ist, und die dritte Basisfunktion $c_{type}(x)$ eine Ausgabe zu der dritten Tageszeit bereitstellt, welche die gleiche oder ungefähr die gleiche für alle Werte der dritten Variable $y$ ist.

**4.** Verfahren nach einem der Ansprüche 1-3, **dadurch gekennzeichnet, dass** die erste Tageszeit entsprechend der lokalen Zeit vor 12 Uhr liegt, und/oder die zweite Tageszeit entsprechend der lokalen Zeit nach 12 Uhr liegt.

**5.** Verfahren nach einem der Ansprüche 1-4, wobei die Lichtausgabe ferner durch eine Intensität gekennzeichnet ist, und der Steuerparameter ferner einen Intensitätssteuerparameter zum Steuern der Intensität der Lichtausgabe umfasst, und das Verfahren ferner umfasst:

**in der ersten Alternative** Bereitstellen einer zweiten Funktion, die umfasst:

eine vierte Variable, welche die lokale Zeit repräsentiert,
einen Bereich von Intensitätssteuerparametern als Ausgabe,
wobei die zweite Funktion eine dritte Spitze und eine vierte Spitze des Bereichs von Intensitätssteuerparametern definiert,
wobei die dritte Spitze ein drittes Maximum zu einer vierten Tageszeit und eine dritte volle Breite bei dem halben Maximum von mindestens 30 Minuten aufweist, und
wobei die vierte Spitze ein viertes Maximum zu einer fünften Tageszeit, die nach der vierten Tageszeit liegt, und eine vierte volle Breite bei dem halben Maximum von mindestens 30 Minute aufweist,

oder **in der zweiten Alternative** Bereitstellen einer zweiten Funktion, repräsentierend:

$$I(t) = I_0(t) \cdot c,$$

wobei $t$ eine vierte Variable ist, welche die lokale Zeit repräsentiert, und die vierte Variable eine Eingabe für die zweite Funktion darstellt,
wobei $I(t)$ die Ausgabe ist, die einen Bereich von Intensitätssteuerparametern abdeckt,
wobei $I_0(t)$ eine vierte Basisfunktion der vierten Variable t ist und eine dritte Spitze und eine vierte Spitze des Bereichs von Farbtemperatursteuerung definiert,
wobei die dritte Spitze ein drittes Maximum zu einer vierten Tageszeit und eine dritte volle Breite bei dem halben Maximum von mindestens 30 Minuten aufweist,
wobei die zweite Spitze ein viertes Maximum zu einer fünften Tageszeit, die nach der vierten Tageszeit liegt, und eine vierte volle Breite bei dem halben Maximum von mindestens 30 Minuten aufweist, und
wobei c ein Korrekturfaktor ist,

und **in sowohl der ersten als auch der zweiten Alternative** Eingeben des aktuellen Zeitparameters als der vierten Variable in die zweite Funktion, um den Intensitätssteuerparameter als die Ausgabe von der zweiten Funktion zu geben.

6. Verfahren nach Anspruch 5, **in der ersten Alternative dadurch gekennzeichnet, dass** die zweite Funktion ein Intensitätsbasisniveau definiert, das eine positive konstante Intensität über einen zweiten Bereich der lokalen Zeit repräsentiert, und die dritte volle Breite bei dem halben Maximum die Breite der dritten Spitze bei einer Intensität entsprechend dem mittleren Wert der Intensität des dritten Maximums und der Intensität auf dem Intensitätsbasisniveau repräsentiert, und/oder die vierte volle Breite bei dem halben Maximum die Breite der vierten Spitze bei einer Intensität entsprechend dem mittleren Wert der Intensität des vierten Maximums und der Intensität auf dem Intensitätsbasisniveau repräsentiert, oder **in der zweiten Alternative dadurch gekennzeichnet, dass** die vierte Basisfunktion $I_0(t)$ gleich oder ungefähr gleich dem Intensitätsbasisniveau über einen zweiten Bereich der lokalen Zeit ist, wobei das Intensitätsbasisniveau eine Konstante ist; wobei die dritte volle Breite bei dem halben Maximum die Breite der dritten Spitze in der Mitte zwischen dem dritten Maximum und dem Intensitätsbasisniveau repräsentiert, und/oder die vierte volle Breite bei dem halben Maximum die Breite der vierten Spitze in der Mitte zwischen dem vierten Maximum und dem Intensitätsbasisniveau repräsentiert.

7. Verfahren nach einem der Ansprüche 5-6, **in der ersten Alternative dadurch gekennzeichnet, dass** die zweite Funktion eine zweite Vertiefung zwischen der dritten Spitze und der vierten Spitze definiert, wobei die zweite Vertiefung ein zweites Minimum zu einer sechsten Tageszeit aufweist, oder **in der zweiten Alternative** dadurch gekennzeichnet, dass die vierte Basisfunktion $I_0(t)$ eine zweite Vertiefung zwischen der dritten Spitze und der vierten Spitze definiert, wobei die zweite Vertiefung ein erstes Minimum zu einer sechsten Tageszeit aufweist.

8. Verfahren nach einem der Ansprüche 5-7, **dadurch gekennzeichnet, dass** die erste Tageszeit und die vierte Tageszeit die gleiche oder ungefähr die gleiche Tageszeit ist, und/oder die zweite Tageszeit und die fünfte Tageszeit die gleiche oder ungefähr die gleiche Tageszeit ist, und/oder die dritte Tageszeit und die sechste Tageszeit die gleiche oder ungefähr die gleiche Tageszeit ist.

9. Verfahren nach einem der Ansprüche, **dadurch gekennzeichnet, dass** das Verfahren ferner umfasst:

   Bereitstellen eines Altersparameters von einem Bereich von Altersparametern, die verschiedene Alter einer Person repräsentieren, und
   wobei **in der ersten Alternative** die zweite Funktion ferner eine multivariable Funktion ist und ferner umfasst:

   eine fünfte Variable, welche den Bereich von Altersparametern als Eingabe repräsentiert, und
   wobei die zweite Funktion zu einem Festwert der vierten Variable eine Ausgabe bereitstellt, die monoton mit der fünften Variable zunimmt,

   oder wobei **in der zweiten Alternative** die zweite Funktion ferner eine multivariable Funktion ist, repräsentierend

$$I(t,\ z) = I_0(t) \cdot c_{age}(z),$$

   wobei $t$ die vierte Variable ist, welche die lokale Zeit repräsentiert, und $z$ eine fünfte Variable ist, die den Bereich von Altersparametern repräsentiert, und die vierte und die fünfte Variable die Eingabe für die zweite Funktion darstellen,
   wobei $I(t, z)$ die Ausgabe der zweiten Funktion ist und den Bereich von Intensitätssteuerparametern abdeckt, wobei $I_0(t)$ die vierte Basisfunktion der vierten Variable $t$ ist, und
   wobei $c_{age}(z)$ eine fünfte Basisfunktion der fünften Variable z ist, wobei die fünfte Basisfunktion den Korrekturfaktor $c$ darstellt und eine Ausgabe bereitstellt, die eine Intensität repräsentiert, die monoton mit dem Alter der Person zunimmt, und wobei **in sowohl der ersten als auch der zweiten Alternative** das Verfahren ferner umfasst:

   Eingeben des Altersparameters für die spezifische Person als der fünften Variable zusätzlich zu dem aktuellen Zeitparameter als der vierten Variable in die zweite Funktion, um den Intensitätssteuerparameter als die Ausgabe von der zweiten Funktion zu geben.

10. Verfahren nach Anspruch 9, **in der ersten Alternative dadurch gekennzeichnet, dass** die zweite Funktion zu einem Festwert der vierten Variable eine Ausgabe bereitstellt, die exponentiell mit der fünften Variable zunimmt, oder **in der zweiten Alternative dadurch gekennzeichnet, dass** die fünfte Basisfunktion $c_{age}(z)$ repräsentiert:

$$c_{age}(z) = 1/(2\wedge(13/(z-25))),$$

wobei der Parameter $z$ in der Einheit von Jahren gegeben ist.

11. Verfahren nach einem der Ansprüche 5-10, **in der ersten Alternative dadurch gekennzeichnet, dass** die zweite Funktion konstant oder ungefähr konstant über einen dritten Bereich der lokalen Zeit zu einem Festwert der fünften Variable ist, und wobei die erste Funktion eine fünfte Spitze mit einem fünften Maximum zu einer siebenten Tageszeit und eine fünfte volle Breite bei dem halben Maximum von mindestens 15 Minuten definiert, und der dritte Bereich der lokalen Zeit nach der zweiten und/oder der vierten Tageszeit liegt und die siebente Tageszeit umfasst, oder **in der zweiten Alternative dadurch gekennzeichnet, dass** die zweite Basisfunktion konstant oder ungefähr konstant über einen dritten Bereich der lokalen Zeit ist, und wobei die erste Basisfunktion eine fünfte Spitze mit einem fünften Maximum zu einer siebenten Tageszeit und eine fünfte volle Breite bei dem halben Maximum von mindestens 15 Minuten definiert, und der dritte Bereich der lokalen Zeit nach der zweiten Tageszeit liegt und die siebente Tageszeit umfasst.

12. Nicht therapeutisches Verfahren zum Bereitstellen von Licht für eine spezifische Person, wobei das Verfahren umfasst:

Bereitstellen einer Lampe, die dazu eingerichtet ist, deren Lichtausgabe durch einen Ausgabesteuerparameter zu variieren, und

Bereitstellen des Ausgabesteuerparameters durch das nicht therapeutische Verfahren nach einem der Ansprüche 1 bis 11.

13. Beleuchtungssystem, das dazu eingerichtet ist, einen Ausgabesteuerparameter mit einer spezifischen Person verbunden zum Steuern einer Lichtausgabe einer Lampe, die dazu eingerichtet ist, die Lichtausgabe durch den Ausgabesteuerparameter zu variieren, bereitzustellen, wobei die Lichtausgabe durch eine Farbtemperatur gekennzeichnet ist, und der Steuerparameter eine Farbtemperatursteuerparameter zum Steuern der Farbtemperatur der Lichtausgabe umfasst, wobei das Beleuchtungssystem umfasst:

eine Berechnungseinheit, die dazu eingerichtet ist, die Ausgabe bereitzustellen, und dazu eingerichtet ist, die folgenden Schritte durch-zuführen:

Bereitstellen eines aktuellen Zeitparameters, der die aktuelle lokale Tageszeit repräsentiert,
Bereitstellen eines Typparameters von einem Bereich von Typparametern umfassend mindestens einen Parameter, der einen ersten Personentyp repräsentiert, und einen Parameter, der einen zweiten Personentyp repräsentiert,
Bereitstellen eines Geschleichtsparameters von einem Bereich von Geschlechtsparametern umfassend einen Parameter, der eine männliche Person repräsentiert, und einen Parameter, der eine weibliche Person repräsentiert, und
**in einer ersten Alternative** Bereitstellen einer ersten Funktion, die eine multivariable Funktion ist und umfassend:

eine erste Variable, welche die lokale Zeit repräsentiert, eine zweite Variable, welche den Bereich von Typparametern repräsentiert, und eine dritte Variable, welche den Bereich von Geschlechtsparametern als Eingabe repräsentiert,
einen Bereich von Farbtemperatursteuerparametern als Ausgabe;
wobei die erste Funktion eine erste Spitze und eine zweite Spitze des Bereichs von Farbtemperatursteuerparametern bezüglich der ersten Variable definiert,
wobei die erste Spitze ein erstes Maximum zu einer ersten Tageszeit und eine erste volle Breite bei dem halben Maximum von mindestens 30 Minuten aufweist,
wobei die zweite Spitze ein zweites Maximum zu einer zweiten Tageszeit, die nach der ersten Tageszeit liegt, und eine zweite volle Breite bei dem halben Maximum von mindestens 30 Minuten aufweist,
wobei die erste Funktion zu einem Festwert der dritten Variable eine Ausgabe bereitstellt, die eine niedrigere Farbtemperatur zu der ersten Tageszeit für den ersten Personentyp als für den zweiten Personentyp repräsentiert, und eine Ausgabe, die eine höhere Farbtemperatur zu der zweiten Tageszeit für den ersten Personentyp als für den zweiten Personentyp repräsentiert, und

wobei die erste Funktion ferner zu einem Festwert der zweiten Variable eine Ausgabe bereitstellt, die eine niedrigere Farbtemperatur zu der ersten Tageszeit und der zweiten Tageszeit für eine männliche Person als für eine weibliche Person repräsentiert,

oder **in einer zweiten Alternative** Bereitstellen einer ersten Funktion, die eine multivariable Funktion ist, repräsentierend:

$$T(t,\ x,\ y) = (T_0(t) - T_b) \cdot c_{type}(x) \cdot c_{gender}(y) + T_b,$$

wobei $t$ eine erste Variable ist, welche die lokale Zeit repräsentiert, $x$ eine zweite Variable ist, welche den Bereich von Typparametern repräsentiert, und $y$ eine dritte Variable ist, welche den Bereich von Geschlechtsparametern repräsentiert, und die erste, die zweite und die dritte Variable eine Eingabe für die erste Funktion darstellen,

wobei $T(t, x, y)$ die Ausgabe der ersten Funktion ist und einen Bereich von Temperatursteuerparametern abdeckt,

wobei $T_0(t)$ eine erste Basisfunktion der ersten Variable $t$ ist und eine erste Spitze und eine zweite Spitze des Bereichs von Farbtemperatursteuerung definiert,

wobei die erste Spitze ein erstes Maximum zu einer ersten Tageszeit und eine volle Breite bei dem halben Maximum von mindestens 30 Minuten aufweist,

wobei die zweite Spitze ein zweites Maximum zu einer zweiten Tageszeit, die nach der ersten Tageszeit liegt, und eine zweite volle Breite bei dem halben Maximum von mindestens 30 Minuten aufweist,

wobei $c_{type}(x)$ eine zweite Basisfunktion der zweiten Variable $x$ ist und eine Ausgabe entsprechend einer niedrigeren Farbtemperatur zu der ersten Tageszeit für den ersten Personentyp als für den zweiten Personentyp bereitstellt, und eine Ausgabe, die eine höhere Farbtemperatur zu der zweiten Tageszeit für den ersten Personentyp als für den zweiten Personentyp repräsentiert,

wobei $c_{gender}(y)$ eine dritte Basisfunktion der dritten Variable $y$ ist und eine Ausgabe entsprechend einer niedrigeren Farbtemperatur zu der ersten Tageszeit und der zweiten Tageszeit für eine männliche Person als für eine weibliche Person bereitstellt, und

wobei $T_b$ ein Farbtemperaturbasisniveau ist, das eine positive Konstante für alle Werte der ersten, der zweiten und der dritten Variable ist,

und **in sowohl der ersten als auch der zweiten Alternative** Eingeben des aktuellen Zeitparameters als der ersten Variable, des Typparameters für die spezifische Person als der zweiten Variable, und des Geschlechtsparameters für die spezifische Person als der dritten Variable in die erste Funktion, um den Farbtemperatursteuerparameter als die Ausgabe von der ersten Funktion zu geben,

wobei das Beleuchtungssystem ferner umfasst:

einen Zeitdetektor oder eine Einheit (51), die mit der Berechnungseinheit verbunden ist zum Bereitstellen der lokalen Tageszeit, und

eine Benutzeroberfläche, die mit der Berechnungseinheit verbunden ist zum Bereitstellen eines Typparameters von einem Bereich von Typparametern umfassend mindestens einen Parameter, der einen ersten Personentyp repräsentiert, und einen Parameter, der einen zweiten Personentyp repräsentiert,

und zum Bereitstellen eines Geschlechtsparameters von einem Bereich von Geschlechtsparametern umfassend einen Parameter, der eine männliche Person repräsentiert, und einen Parameter, der eine weibliche Person repräsentiert.

14. Beleuchtungssystem nach Anspruch 13, **dadurch gekennzeichnet, dass** die Benutzeroberfläche ferner für manuelles Bereitstellen eines Altersparameters von einem Bereich von Altersparametern bestimmt ist, die verschiedene Alter einer Person repräsentieren.

15. Vorrichtung nach Anspruch 13, die dazu eingerichtet ist, eine Implementierung des Verfahrens nach einem der Ansprüche 1-12 für nicht therapeutischen Einsatz durchzuführen.

16. Kit umfassend eine Lampe, die dazu eingerichtet ist, ihre Lichtausgabe durch einen Ausgabesteuerparameter zu variieren, und Beleuchtungssystem nach einem der Ansprüche 13 bis 16 zum Bereitstellen des Ausgabesteuerparameters zum Steuern der Lichtausgabe der Lampe, wobei das Beleuchtungssystem ferner mit der Lampe verbunden ist, um den Ausgabesteuerparameter zu der Lampe zu übertragen.

**Revendications**

1. Procédé non thérapeutique pour fournir un paramètre de contrôle de sortie associé avec une personne spécifique pour contrôler une émission de lumière d'une lampe adaptée pour varier ladite émission de lumière par ledit paramètre de contrôle de sortie, ladite émission de lumière étant caractérisée par une température de couleur et ledit paramètre de contrôle comprenant un paramètre de contrôle de température pour contrôler la température de couleur de ladite émission de lumière, ledit procédé comprenant:

la fourniture d'un paramètre d'heure actuelle représentant l'heure actuelle locale du jour,
la fourniture d'un paramètre type d'une gamme de paramètres types comprenant au moins un paramètre représentant une premier catégorie de personne et un paramètre représentant une deuxième catégorie de personne,
la fourniture d'un paramètre de sexe à partir d'une gamme de paramètres de sexe comprenant un paramètre représentant une personne de sexe masculin et un paramètre représentant une personne de sexe féminin, et **dans une première alternative,** fournissant une première fonction étant une fonction multivariable et comprenant:

une première variable représentant l'heure locale, une deuxième variable représentant ladite gamme de paramètres types, et une troisième variable représentant ladite gamme de paramètres de sexe en tant qu'entrée,
une gamme de paramètres de contrôle de température de couleur en tant que sortie;
ladite première fonction définissant une premi ère pointe et une deuxième pointe de ladite gamme de paramètres de contrôle de température de couleur par rapport à ladite première variable,
ladite première pointe ayant un premier maximum à une première heure du jour et une première largeur complète à la moitié du maximum d'au moins 30 minutes,
ladite deuxième pointe ayant un deuxième maximum à une deuxième heure du jour qui survient après ladite première heure du jour et une deuxième largeur complète à la moitié du maximum d'au moins 30 minutes,
ladite première fonction fournissant, à une valeur fixe de ladite troisième variable, une sortie représentant une température de couleur inférieure à ladite première heure du jour pour ladite première catégorie de personne que pour ladite deuxième catégorie de personne, et une sortie représentant une température de couleur supérieure à ladite deuxième heure du jour pour ladite première catégorie de personne que pour ladite deuxième catégorie de personne, et
ladite première fonction fournissant en outre, à une valeur fixe de ladite deuxième variable, une sortie représentant une température de couleur inférieure à ladite première heure du jour et ladite deuxième heure du jour pour une personne de sexe masculin que pour une personne de sexe féminin,

ou, **dans une deuxième alternative,** fournissant une première fonction étant une fonction multivariable représentant:

$$T(t,\ x,\ y) = (T_0(t) - T_b) \cdot c_{type}(x) \cdot c_{gender}(y) + T_b,$$

où $t$ est une première variable représentant l'heure locale, $x$ est une deuxième variable représentant ladite gamme de paramètres types, et $y$ est une troisième variable représentant ladite gamme de paramètres de sexe, et lesdites première, deuxième et troisième variables constituent une entrée pour ladite première fonction,
$T(t,\ x,\ y)$ est la sortie de ladite première fonction et couvre une gamme de paramètres de contrôle de température,
$T_0(t)$ est une première fonction de base de ladite première variable $t$ est définissant une première pointe et une deuxième pointe de ladite gamme de contrôle de température de couleur,
ladite première pointe ayant un premier maximum à une première heure du jour et une première largeur complète à la moitié du maximum d'au moins 30 minutes,
ladite deuxième pointe ayant un deuxième maximum à une deuxième heure du jour qui survient après ladite première heure du jour et une deuxième largeur complète à la moitié du maximum d'au moins 30 minutes,

$c_{type}(x)$ est une deuxième fonction de base de la deuxième variable $x$ est fournissant une sortie correspondant à une température de couleur inférieure à ladite première heure du jour pour ladite première catégorie de personne que pour ladite deuxième catégorie de personne et une sortie représentant une température de couleur supérieure à ladite deuxième heure du jour pour ladite première catégorie de personne que pour ladite deuxième catégorie de personne,

$c_{gender}(y)$ est une troisième fonction de base de ladite troisième variable $y$ et fournissant une sortie correspondant à une température de couleur inférieure à ladite première heure du jour et ladite deuxième heure du jour pour une personne de sexe masculin que pour une personne de sexe féminin, et

$T_b$ est un niveau de base de température de couleur qui est une constante positive pour toutes les valeurs desdites premiére, deuxième, et troisième variables,

et, **dans autant la première que la deuxième alternative,** entrant dans ladite première fonction ledit premier paramètre d'heure actuelle en tant que ladite première variable, ledit paramètre type pour ladite personne spécifique en tant que ladite deuxième variable, et ledit paramètre de sexe pour ladite personne spécifique en tant que ladite troisième variable pour générer ledit paramètre de contrôle de température de couleur en tant que la sortie de ladite première fonction.

2. Procédé selon la revendication 1 caractérisé, **dans la première alternative,** en ce que ladite première fonction définissant un niveau de base de température de couleur représentant une température de couleur constante positive sur une première gamme de l'heure locale, et ladite température de couleur étant indépendante de ladite deuxième variable et ladite troisième variable sur ladite première gamme, et ladite première largeur complète à la moitié du maximum représentant la largeur de ladite première pointe à une température de couleur correspondant à la moyenne de la température de couleur dudit premier maximum et la température de couleur audit niveau de base de température de couleur, et ladite deuxième largeur complète à la moitié du maximum représentant la largeur de ladite deuxième pointe à une température de couleur correspondant à la moyenne de la température de couleur dudit deuxième maximum et la température de couleur audit niveau de base de température de couleur, ou caractérisé, **dans la deuxième alternative** en ce que ladite première fonction de base $T_0(t)$ est zéro ou environ zéro sur une première gamme de l'heure locale et ladite première largeur complète à ladite moitié du maximum représentant la largeur de ladite première pointe au milieu entre ledit premier maximum et $T_b$, et ladite deuxième largeur complète à la moitié du maximum représentant la largeur de ladite deuxième pointe au milieu entre ledit deuxième maximum et $T_b$.

3. Procédé selon l'une quelconque des revendications 1 à 2 caractérisé, **dans la première alternative,** en ce que ladite première fonction définissant une première dépression entre ladite première pointe et ladite deuxième pointe à des valeurs fixes desdites deuxième et troisième variables, ladite première dépression ayant un premier minimum à une troisième heure du jour et ladite première fonction fournissant une sortie à ladite troisième heure du jour qui est la même ou environ la même pour toutes les valeurs de ladite deuxième variable et ladite troisième variable, ou, **dans la deuxième alternative, caractérisé en ce que** ladite première fonction de base $T_0(t)$ définissant une première dépression entre ladite première pointe et ladite deuxième pointe, ladite première dépression ayant un premier minimum à une troisième heure du jour, ladite deuxième fonction de base $c_{type}(x)$ fournissant une sortie à ladite troisième heure du jour qui est le même ou environ le même pour toutes les valeurs de ladite deuxième variable $x$, et ladite troisième fonction de base $c_{type}(x)$ fournissant une sortie à ladite troisième heure du jour qui est le même ou environ le même pour toutes les valeurs de ladite troisième variable $y$.

4. Procédé selon l'une quelconque des revendications 1 à 3, **caractérisé en ce que** ladite première heure du jour étant avant midi selon l'heure locale et/ou ladite deuxième heure du jour étant après midi selon l'heure locale.

5. Procédé selon l'une quelconque des revendications 1 à 4, ladite émission de lumière étant en outre **caractérisée par** une intensité et ledit paramètre de contrôle comprenant en outre un paramètre de contrôle d'intensité pour contrôler l'intensité de ladite émission de lumière, et ledit procédé comprenant en outre:

**dans la première alternative,** fournissant une deuxième fonction comprenant:

une quatrième variable représentant l'heure locale,
une gamme de paramètres de contrôle d'intensité en tant que sortie,
ladite deuxième fonction définissant une troisième pointe et une quatrième pointe de ladite gamme de paramètres de contrôle d'intensité,

ladite troisième pointe ayant un troisième maximum à une quatrième heure du jour et une troisième largeur complète à la moitié du maximum d'au moins 30 minutes, et

ladite quatrième pointe ayant un quatrième maximum à une cinquième heure du jour qui survient après ladite quatrième heure du jour et une quatrième largeur complète à la moitié du maximum d'au moins 30 minutes,

ou, **dans ladite deuxième alternative,** fournissant une deuxième fonction représentant:

$$I(t) = I_0(t) \cdot c,$$

où $t$ est une quatrième variable représentant l'heure locale et ladite quatrième variable constitue une entrée pour ladite deuxième fonction,

$I(t)$ est la sortie couvrant unre gamme de paramètres de contrôle d'intensité,

$I_0(t)$ est une quatrième function de base d'une quatrième variable $t$ et définissant une troisième pointe et une quatrième pointe de ladite gamme de contrôle de température de couleur,

ladite troisième pointe ayant un troisième maximum à une quatrième heure du jour et une troisième largeur complète à la moitié du maximum d'au moins 30 minutes,

ladite deuxième pointe ayant un quatrième maximum à une cinquième heure du jour qui survient après ladite quatrième heure du jour et une quatrième largeur complète à la moitié du maximum d'au moins 30 minutes, et

$c$ est un facteur de correction,

et, **dans autamt la première que la deuxième alternative** laissant entrer dans ladite deuxième fonction ledit paramètre d'heure actuelle en tant que ladite quatrième variable afin de générer ledit paramètre de contrôle d'intensité en tant que sortie de ladite deuxième fonction.

6. Procédé selon la revendication 5, **dans la première alternative caractérisé en ce que** ladite deuxième fonction définissant un niveau de base d'intensité représentant une intensité constante positive sur une deuxième gamme de l'heure locale, et ladite troisième largeur complète à la moitié du maximum représentant la largeur de ladite troisième pointe à une intensité correspondant à la moyenne de l'intensité dudit troisième maximum et l'intensité audit niveau de base d'intensité, et/ou ladite quatrième largeur complète à la moitié du maximum représentant la largeur de ladite quatrième pointe à une intensité correspondant à la moyenne de l'intensité dudit quatrième maximum et l'intensité audit niveau de base d'intensité, ou caractérisé, **dans la deuxième alternative,** par ladite quatrième fonction de base $I_0(t)$ étant égale à ou environ égale à un niveau de base d'intensité sur une deuxiéme gamme de l'heure locale où ledit niveau de base d'intensité est une constante, ladite troisième largeur complète à la moitié du maximum représentant la largeur de ladite troisième pointe au milieu entre ledit troisième mximum et ledit niveau de base d'intensité, et/ou ladite quatrième largeur complète à la moitié du maximum représentant la largeur de ladite quatrième pointe au milieu entre ledit quatrième maximum et ledit niveau de base d'intensité.

7. Procédé selon l'une quelconque des revendications 5 à 6, caractérisé, **dans la première alternative,** en ce que la deuxième fonction définissant une deuxième dépression entre ladite troisième pointe et ladite quatrième pointe, ladite deuxième dépression ayant un deuxième minimum à une sixième heure du jour, ou caractérisé, **dans la deuxième alternative,** en ce que la quatrième fonction de base $I_0(t)$ définissant une deuxième dépression entre ladite troisième pointe et ladite quatrième pointe, ladite deuxième dépression ayant un premier minimum à une sixième heure du jour.

8. Procédé selon l'une quelconque des revendications 5 à 7, **caractérisé en ce que** ladite première heure du jour et ladite quatrième heure du jour étant la même ou environ la même heure du jour, et/ou ladite deuxième heure du jour et ladite cinquième heure du jour étant la même ou environ la même heure du jour, et/ou ladite troisième heure du jour et ladite sixième heure du jour étant la même ou environ la même heure du jour.

9. Procédé selon l'une quelconque des revendications 5 à 8, **caractérisé en ce que** ledit procédé comprenant en outre:

la fourniture d'un paramètre d'âge à partir d'une gamme de paramètres d'âge représentant des âges différents d'une personne, et

**dans la première alternative,** ladite deuxième fonction étant en outre une fonction multivariable et comprenant en outre:

une cinquième variable représentant ladite gamme de paramètres d'âge en tant qu'entrée, et
ladite deuxième fonction fournissant, à une valeur fixée de ladite quatrième variable, une sortie qui augmente de manière monotone avec ladite cinquième variable,

ou, **dans la deuxième alternative,** ladite deuxième fonction étant en outre une fonction multivariable représentant

$$I(t, z) = I_0(t) \cdot c_{age}(z),$$

où $t$ est ladite quatrième variable représentant l'heure locale, et $z$ est une cinquième variable représentant ladite gamme de paramètres d'âge, et lesdites quatrième et cinquième variables constituent ladite entrée pour ladite deuxième fonction,
$I(t, z)$ est la sortie de ladite deuxième fonction et couvre ladite gamme de paramètres de contrôle d'intensité,
$I_0(t)$ est ladite quatrième fonction de base de ladite quatrième variable $t$, et
$c_{age}(z)$ est une cinquième fonction de base de ladite cinquième variable $z$, ladite cinquième fonction de base constituant ledit facteur de correction $c$ et fournissant une sortie représentant une intensité qui augmente de manière monotone avec l'âge de ladite personne, et dans **à la fois la première que la deuxième alternative** ledit procédé comprend en outre:

entrant dans ladite deuxième fonction ledit paramètre d'âge pour ladite personne specifique en tant que ladite cinquième variable en complément audit paramètre d'heure actuelle en tant que ladite quatrième variable afin de générer ledit paramètre de contrôle d'intensité en tant que sortie de ladite deuxième fonction.

10. Procédé selon la revendication 9 caractérisé, **dans la première alternative,** en ce que ladite deuxième fonction fournit, à une valeur fixée de ladite quatrième variable, une sortie qui augmente de manière exponentielle avec ladite cinquième variable, ou caractérisé, **dans la deuxième alternative,** en ce que ladite cinquième fonction de base $c_{age}(z)$ représentant:

$$c_{age}(z) = 1/(2^{\wedge}(13/(z-25))),$$

où le paramètre z est donné dans l'unité d'années.

11. Procédé selon l'une quelconque des revendications 5 à 10 caractérisé, **dans la première alternative,** en ce que ladite deuxième fonction étant constante ou environ constante sur une troisième gamme de l'heure locale à une valeur fixée de ladite cinquième variable, et ladite première fonction définissant une cinquième pointe ayant un cinquième maximum à une septième heure du jour et une cinquième largeur complète à la moitié du maximum d'au moins 15 minutes, et ladite troisième gamme de l'heure locale étant après ladite deuxième et/ou ladite quatrième heure du jour et comprenant ladite septième heure du jour, ou caractérisé, **dans une deuxième alternative,** en ce que ladite deuxième fonction de base étant constante ou environ constante sur une troisième gamme de l'heure locale et ladite première fonction de base définissant une cinquième pointe ayant un cinquième maximum à une septième heure du jour et eun cinquième largeur complète à la moitié du maximum d'au moins 15 minutes, et ladite troisième gamme de l'heure locale étant après ladite deuxième heure du jour et comprenant ladite septième heure du jour.

12. Procédé non thérapeutique pour fournir de la lumière pour une personne spécifique, ledit procédé comprenant:

la fourniture d'une lampe adaptée pour varier son émission de lumière par un paramètre de contrôle, et
la fourniture dudit paramètre de contrôle de sortie par le procédé non thérapeutique selon l'une quelconque des revendications 1 à 11.

13. Système d'éclairage adapté pour fournir un paramètre de contrôle de sortie associé avec une personne spécifique pour contrôler une émission de lumière d'une lampe adaptée pour varier ladite émission de lumière par ledit paramètre de contrôle de sortie, ladite émission de lumière étant **caractérisée par** une température de couleur et ledit paramètre de contrôle comprenant un paramètre de contrôle de température de couleur pour contrôler la termpérature de couleur de ladite émission de lumière, ledit système d'éclairage comprenant:

une unité de calcul adaptée pour fournir ladite sortie et adaptée pour effectuer les étapes suivantes:

la fourniture d'un paramètre d'heure actuelle représentant l'heure locale actuelle du jour,
la fourniture d'un paramètre type à partir d'une gamme de paramètres types comprenant au moins un paramètre représentant une première catégorie de personne et un paramètre représentant une deuxième catégorie de personne,
la fourniture d'un paramètre de sexe à partir d'une gamme de paramètres de sexe comprenant un paramètre représentant une personne de sexe masculin et un paramètre représentant une personne de sexe féminin, et
**dans une première alternative,** fournir une première fonction étant une fonction multivariable et comprenant:

une premiére variable représentant l'heure locale, une deuxième variable représentant ladite gamme de paramètres types, et une troisième variable représentant ladite gamme de paramètres de sexe en tant qu'entrée,
une gamme de paramètres de contrôle de température de couleur en tant que sortie;
ladite première fonction définissant une première pointe et une deuxième pointe de ladite gamme de paramètres de contrôle de température de couleur par rapport à ladite première variable,
ladite première pointe ayant un premier maximum à une première heure du jour et une première largeur complète à la moitié du maximum d'au moins 30 minutes,
ladite deuxième pointe ayant un deuxième maximum à une deuxième heure du jour qui survient après ladite première heure du jour et une deuxième largeur complète à la moitié du maximum d'au moins 30 minutes,
ladite première fonction fournissant à une valeur fixée de ladite troisième variable une sortie représentant une température de couleur inférieure à ladite première heure du jour pour ladite première catégorie de personne que pour ladite deuxième catégorie de personne et une sortie représentant une température de couleur supérieure à ladite deuxième heure du jour pour ladite premiére catégorie de personne que pour ladite deuxième type, et
ladite première fonction fournissant en outre à une valeur fixée de ladite deuxième variable une sortie représentant une température de couleur inférieure à ladite première heure du jour et ladite deuxième heure du jour pour une personne de sexe masculin que pour une personne de sexe feminin,
ou, **dans une deuxième alternative,** fournissant une première fonction étant une fonction multivariable représentant:

$$T(t,\ x,\ y) = (T_0(t) - T_b) \cdot c_{type}(x) \cdot c_{gender}(y) + T_b,$$

où $t$ est une première variable représentant l'heure locale, $x$ est une deuxième variable représentant ladite gamme de paramètres types, et $y$ est une troisième variable représentant ladite gamme de paramètres de sexe, et lesdites première, deuxième et trosième variables constituent une entrée pour ladite première fonction,
$T(t,\ x,\ y)$ est la sortie de ladite première fonction et couvre une gamme de paramètres de contrôle de température,
$(T_0(t)$ est une première fonction de base de la première variable $t$ et définissant une première pointe et une deuxième pointe de ladite gamme de contrôle de température de couleur,
ladite première pointe ayant un premier maximum à une première heure du jour et une première largeur complète à la moitié du maximum d'au moins 30 minutes,
ladite deuxième pointe ayant un deuxième maximum à une deuxième heure du jour qui survient après la première heure du jour et une deuxième largeur complète à la moitié du maximum d'au moins 30 minutes,
$c_{type}(x)$ est une deuxième fonction de base de la deuxième variable $x$ et fournissant une sortie correspondant à une température de couleur inférieure à ladite première heure du jour pour ladite première catégorie de personne que pour ladite deuxième catégorie de personne, et une sortie représentant une température de couleur supérieure à ladite deuxième heure du jour pour ladite première catégorie de personne que pour ladite deuxième catégorie de personne,
$c_{gender}(y)$ est une troisième fonction de base de la troisième variable $y$ est fournissant une sortie correspondant à une température de couleur inférieure à ladite première heure du jour et ladite deuxième heure du jour pour une personne de sexe masculin que pour une personne de sexe féminin, et
$T_b$ est un niveau de base de température de couleur qui est une constante positive pour toutes les valeurs desdites première, deuxième et troisième variables,

et **dans autant la première que la deuxième alternative,** entrant dans ladite première fonction ledit paramètre d'heure actuelle en tant que ladite première variable, ledit paramètre type pour ladite personne spécifique en tant que deuxième variable, et ledit paramètre de sexe pour ladite personne spécifique en tant que ladite troisième variable afin de générer ledit paramètre de contrôle de température de couleur en tant que la sortie de ladite première fonction,
ledit système d'éclairage comprenant en outre:

un détecteur d'heure ou une unité relié(e) à ladite unité de calcul pour fournir ladite heure locale du jour; et

une interface utilisateur reliée à ladite unité de calcul pour fournir un paramètre type à partir d'une gamme de paramètres types comprenant au moins un paramètre représentant une première catégorie de personne et un paramètre représentant une deuxième catégorie de personne,

et pour fournir un

paramètre de sexe à partir d'une gamme de paramètres de sexe comprenant un paramètre représentant une personne de sexe masculin et un paramètre représentant une personne de sexe féminin.

14. Système d'éclairage selon la revendication 13, **caractérisé en ce que** ladite interface utilisateur existant en outre pour fournir manuellement un paramèter d'âge à partir d'une gamme de paramètres représentant des âges différents d'une personne.

15. Appareil selon la revendication 13, étant adapté pour effectuer une mise en oeuvre du procédé selon l'une quelconque des revendications 1 à 12 pour application non thérapeutique ou thérapeutique.

16. Ensemble comprenant une lampe adaptée pour varier son émission de lumière par un paramètre de contrôle de sortie et un système d'éclairage selon l'une quelconque des revendications 13 à 15 pour fournir ledit paramètre de contrôle de sortie pour contrôler ladite émission de lumière de ladite lampe, ledit système d'éclairage étant en outre lié à ladite lampe pour communiquer ledit paramètre de contrôle de sortie à ladite lampe.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

1

LIGHTING
CONTROL
SOFTWARE

Background Light

COMPU-
TATIONAL
UNIT

LIGHT
SENSOR

9

206    12    206    202    203

212

205

9'

207

10

8

FIG. 14

201

202

207

214

213

201

202

214

FIG. 15

203

230

231

232

232

FIG. 16

FIG. 17

FIG. 18

EP 2 988 823 B1

FIG. 19

FIG. 20

FIG. 21A

FIG. 21B

FIG. 22

FIG. 23

**EP 2 988 823 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050015122 A

- WO 2012146256 A